# EUROPEAN PATENT SPECIFICATION

(11) **EP 3 746 441 B1**
(45) Date of publication and mention of the grant of the patent: **05.04.2023**
(21) Application number: 19706097.3
(22) Date of filing: 01.02.2019
(51) Int. Cl.: C07D 471/04, A61K 31/437, A61P 11/00

(54) **PHARMACEUTICAL COMPOUND, SALTS THEREOF, FORMULATIONS THEREOF, AND METHODS OF MAKING AND USING SAME**
PHARMAZEUTISCHE VERBINDUNG, IHRE SALZE, FORMULIERUNGEN DAVON UND VERFAHREN ZUR HERSTELLUNG UND VERWENDUNG DAVON
COMPOSÉ PHARMACEUTIQUE, SELS DE CELUI-CI, FORMULATIONS DE CELUI-CI, ET PROCÉDÉS DE FABRICATION ET D'UTILISATION DE CELUI-CI

(30) Priority: 02.02.2018 WO PCT/CN2018/075023
(43) Date of publication of application: 09.12.2020
(73) Proprietor: GENENTECH, INC., South San Francisco, CA 94080 (US)
(72) Inventor: NAGAPUDI, Karthik, South San Francisco, CA 94080 (US); LIU, Yuan, Princeton, NJ 08540 (US); WANG, Shuai, Princeton, NJ 08540 (US); ZHANG, Wei, South San Francisco, CA 94080-4990 (US); BLATTER, Fritz, 4002 Basel (CH); SASTRY, Srikonda, South San Francisco, CA 94080-4990 (US); LEUNG, Manshiu, South San Francisco, CA 94080-4990 (US); RADHAKRISHNAN, Ramachandran, South San Francisco, CA 94080-4990 (US); TANDALE, Rajendra, S., Stockton, CA 95215 (US); PILSL, Ludwig, Parsippany, NJ 07054 (US); MULLER, Roland, Parsippany, NJ 07054 (US); FRIESER, Markus, Parsippany, NJ 07054 (US); CZAUDERNA, Christine, Parsippany, NJ 07054 (US); FISHER, Lawrence Emerson, South San Francisco, CA 94080-4990 (US)
(74) Representative: Arth, Hans-Lothar
(86) International application number: PCT/US2019/016386
(87) International publication number: WO 2019/152863

(56) References cited:
- WO-A1-2014/055548
- WO-A1-2015/153683

## Description

### CROSS REFERENCE TO RELATED APPLICATIONS

Priority is claimed to PCT/CN2018/075023, filed February 2, 2018.

### BACKGROUND

### Field of the Disclosure

The subject-matter for which protection is sought is as defined by the claims. Any reference to a "disclosure" not falling under the scope of the claims serves explanatory purposes and does not form part of the invention. Any reference to a method of treatment is to be interpreted as a reference to the compounds, pharmaceutical compositions and medicaments of the present invention for use in a method for treatment of the human (or animal) body by therapy (or for diagnosis). The disclosure relates generally to Compound I described herein, novel salts, hydrates, and solvates thereof, and methods of making and using the same. More particularly, the disclosure relates to various salts, including tosylate salts, of Compound I, pharmaceutical and diagnostic compositions containing them, and to their medical use, particularly as drugs in the treatment and/or prevention of diseases.

### Brief Description of Related Technology

Compound I is an orally available small molecule having the structure:

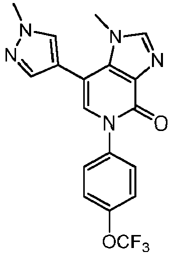

WO 2015/153683 and WO 2014/055548 disclose compound I free base and compound I hydrochloride (see [0235] and [0232] respectively). Compound I has therapeutic value in several different indications that display fibrotic pathophysiology, including idiopathic pulmonary fibrosis (IPF).

Idiopathic pulmonary fibrosis is a disease of unknown etiology that occurs mainly in middle-aged and elderly patients, which is characterized by progressive fibrosis of the lung, leading to pulmonary insufficiency and death. Because fibrosis has long been considered to be a clinically irreversible process, treatments have traditionally been focused on managing the symptoms and complications, with little hope of significantly slowing progression of the condition. For many years, mainstay treatments have been typically antiinflammatory, immunosuppressive, and anti-oxidant agents. The effectiveness of these therapies in the treatment of IPF and other fibrotic conditions appears to be minimal and variable, and their side effects are often poorly tolerated by patients.

New treatment options have only recently become available. Both pirfenidone and nintedanib have been approved for use in the treatment of IPF. Current research efforts to develop new anti-fibrotic agents are targeting multiple mechanisms proposed to be linked to the underlying molecular pathogenic processes. This changing landscape has raised hopes and expectations for what might be achievable with new single agents or combination therapies targeting additional pathways.

### SUMMARY

The present invention relates to a salts, hydrates, and solvates of Compound I. In one aspect, the invention relates to tosylate salts of Compound I. Optionally, the salt is not a hydrochloride salt of Compound I. In another aspect, the invention relates to a pharmaceutical composition including Compound I or a salt, hydrate, or solvate thereof and a pharmaceutically acceptable excipient or carrier. Another aspect of the disclosure provides for a process of formulating pharmaceutical compositions including Compound I and salts, hydrates, or solvates thereof into oral dosage forms.

Some embodiments of the present disclosure relate to methods of administering Compound I or a salt, hydrate, or solvate thereof to treat a patient suffering from a fibrotic disorder, an inflammatory disorder, or an autoimmune disorder, including administering a therapeutically effective amount of Compound I or a salt, hydrate, or solvate thereof.

Some embodiments of the present disclosure relate to methods of administering Compound I or a salt, hydrate, or solvate thereof to treat a subject suffering from a fibrotic disorder, an inflammatory disorder, or an autoimmune disorder, including: administering to a subject a therapeutically effective amount of Compound I or a salt, hydrate, or solvate thereof, wherein the bioavailability of Compound I is equal or increased compared to the bioavailability of the same amount of Compound I administered as a nanosuspension.

Another aspect of the disclosure provides a method for synthesizing Compound I and salts, hydrates, and solvates thereof. Another aspect of the disclosure provides a method for the synthesis of Compound I in a methanol-free process. Yet another aspect of the disclosure provides a method for the synthesis of Compound I tosylate salts in a methanol-free process.

Some additional embodiments of the present disclosure relate to kits including a pharmaceutical composition, prescribing information, and a container, wherein the pharmaceutical composition comprises a therapeutically effective amount of Compound I.

In any embodiment of the methods or kits described herein, the effective daily amount of Compound I is from about 1 mg to about 5000 mg per day, about 5 mg to about 2500 mg per day, or about 10 mg to about 2000 mg per day, on a free base basis. In some further embodiments, the amount of Compound I administered is from about 25 mg to about 1600 mg per day, on a free base basis. In some further embodiments, the amount of Compound I administered is about 25 mg, about 75 mg, about 200 mg, about 275 mg, about 400 mg, about 550 mg, about 575 mg, about 800 mg, about 1150 mg, or about 1600 mg per day, on a free base basis, or in a range defined by any two of the preceding values.

In any embodiment of the methods described herein, the subject treated is suffering from a disease or condition described herein, for example a fibrotic disorder, or specifically idiopathic pulmonary fibrosis (IPF).

For the compositions and methods described herein, optional features, including but not limited to components, compositional ranges thereof, substituents, conditions, and steps, are contemplated to be selected from the various aspects, embodiments, and examples provided herein.

Further aspects and advantages will be apparent to those of ordinary skill in the art from a review of the following detailed description, taken in conjunction with the drawings. While the compounds, compositions, and methods are susceptible of embodiments in various forms, the description hereafter includes specific embodiments with the understanding that the disclosure is illustrative, and is not intended to limit the invention to the specific embodiments described herein.

### BRIEF DESCRIPTION OF THE DRAWINGS

For further facilitating the understanding of the present invention, fifteen drawing figures are appended hereto.
Figure 1 shows an XRPD trace of Compound I, Form A (free base).
Figure 2 shows XRPD traces of Compound I HCl salts: amorphous mono-HCl (bottom trace), crystalline di-HCl (middle trace), and crystalline mono-HCl (top trace).
Figure 3 shows XRPD traces of Compound I HBr salts: amorphous mono-HBr (bottom trace), crystalline mono-HBr (middle trace) and crystalline di-HBr (top trace).
Figure 4 shows XRPD traces of Compound I sulfate salt.
Figure 5 shows XRPD traces of Compound I amorphous nitrate salt (bottom trace) and crystalline nitrate salt (top trace).
Figure 6 shows an XRPD trace of Compound 1 besylate salt.
Figure 7 shows an XRPD trace of Compound 1 hemi-edisylate salt.
Figure 8 shows XRPD traces of Compound I mono-tosylate salt.
Figure 9 shows XRPD traces of Compound I free base (bottom trace), and Compound I di-tosylate salt (middle and top traces).
Figure 10 shows XRPD traces of Compound I free base (bottom trace), and Compound I hemi-napadisylate salt (middle and top traces).
Figure 11 shows XRPD traces of a mixture of Compound I phosphate Type A and Compound I free base type A (bottom trace) and Compound I free base Type A (top trace).
Figure 12 shows XRPD traces of Compound I mesylate (Type A, bottom trace) and Compound I free base (Type A, top trace).
Figure 13 shows the mean plasma concentrations of Compound I following a single 200 mg tablet oral dose of Compound I to male beagle dogs in four formulations including a nanosuspension of the free base, jet-milled free base, amorphous free base, or the mono-tosylate salt.
Figure 14 shows the mean plasma concentrations of Compound I following a single 200 mg tablet oral dose of Compound I to male beagle dogs as a nanosuspension formulation or as the mono-tosylate salt.
Figure 15 shows the mean plasma concentrations of Compound I following a single 200 mg tablet oral dose of Compound I to male beagle dogs as the mono- or di-tosylate salt.
Figure 16 shows the effect of HPMC in maintaining supersaturation of Compound I after its mono-tosylate salt dissolves.
Figure 17 shows the particle size distribution of Compound I mono-tosylate salt produced by antisolvent addition over 6 hours.
Figure 18 shows the particle size distribution of Compound I mono-tosylate salt produced by antisolvent addition over 2 hours.

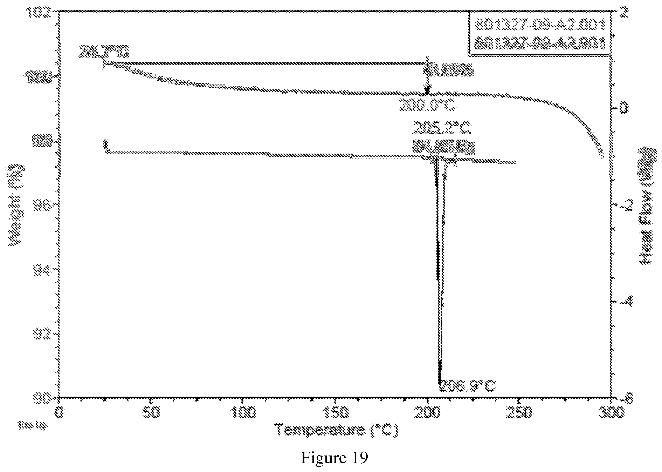

Figure 19 shows the TGA and DSC curves for Compound I mono-tosylate salt.

### DETAILED DESCRIPTION

The details of one or more embodiments of the disclosure are set forth in the accompanying description below. Although any methods and materials similar or equivalent to those described herein can be used in the practice or testing of the present invention, the preferred methods and materials are now described. Other features, objects, and advantages of the disclosure will be apparent from the description. In the specification, the singular forms also include the plural unless the context clearly dictates otherwise. Unless defined otherwise, all technical and scientific terms used herein have the same meaning as commonly understood by one of ordinary skill in the art to which this invention belongs. In the case of conflict, the present specification will control. The compounds and method are contemplated to include embodiments including any combination of one or more of the additional optional elements, features, and steps further described below (including those shown in the figures), unless stated otherwise.

In jurisdictions that forbid the patenting of methods that are practiced on the human body, the meaning of "administering" of a composition to a human subject shall be restricted to prescribing a controlled substance that a human subject will self-administer by any technique (e.g., orally, inhalation, topical application, injection, insertion, etc.). The broadest reasonable interpretation that is consistent with laws or regulations defining patentable subject matter is intended. In jurisdictions that do not forbid the patenting of methods that are practiced on the human body, the "administering" of compositions includes both methods practiced on the human body and also the foregoing activities.

As used herein, the term wt.% refers to percentage by weight based on the total weight.

The section headings used herein are for organizational purposes only and are not to be construed as limiting the subject matter described.

Provided below are salts of Compound I (formula C₁₈H₁₄N₅O₂F₃, mass 389.3 g/mol), having the structure shown below.

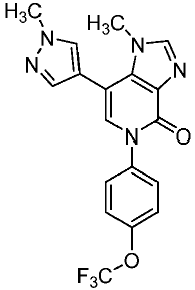

The salt can be selected from the group consisting of besylate, citrate, fumarate, hemi-edisylate, hemi-napadisylate, hydrobromide, maleate, nicotinate, nitrate, oxalate, phosphate, saccharinate, sulfate, L-tartrate, and tosylate salts of Compound I. Optionally, the salt can be selected from besylate, hemi-edisylate, hemi-napadisylate, hydrobromide, nitrate, phosphate, sulfate, and tosylate salts of Compound I. For example, the salt can be a tosylate salt, e.g. a mono-tosylate or a di-tosylate. In one type of embodiment contemplated, the salt is a mono-tosylate salt of Compound I. In another type of embodiment contemplated, the salt is a di-hydrochloride salt of Compound I.

In one class of embodiments, the di-hydrochloride salt of Compound I is amorphous. In another class of embodiments, the di-hydrochloride salt of Compound I is crystalline. In another class of embodiments, there is a composition which includes a mixture of amorphous and crystalline di-hydrochloride salts of Compound I.

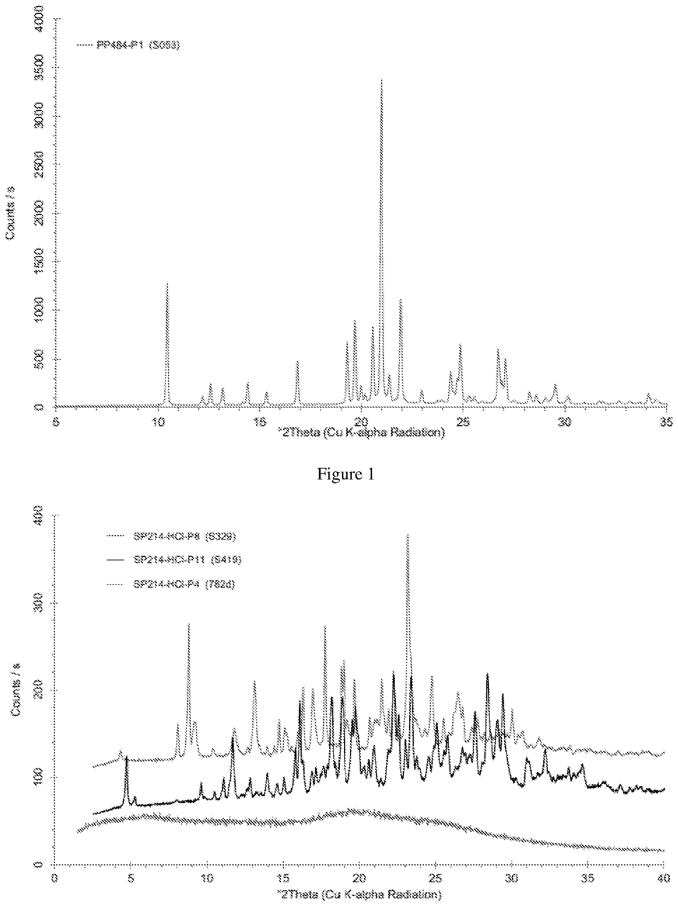

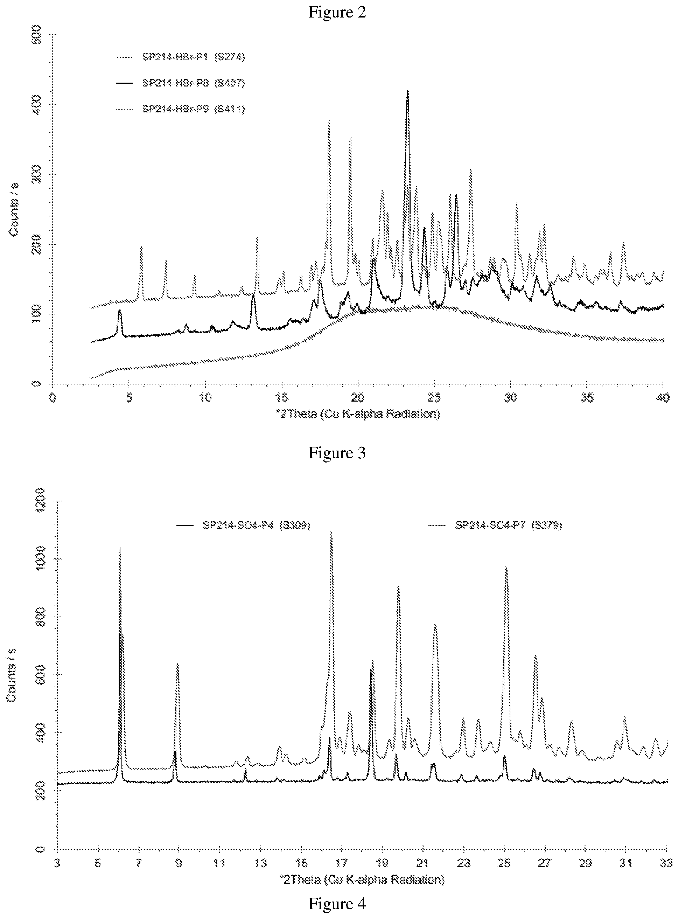

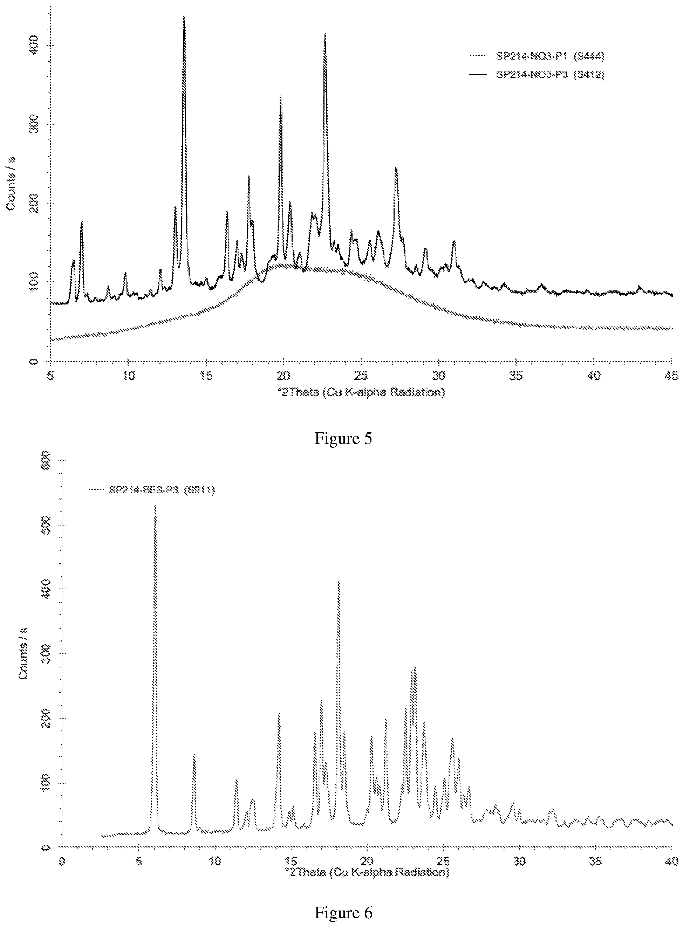

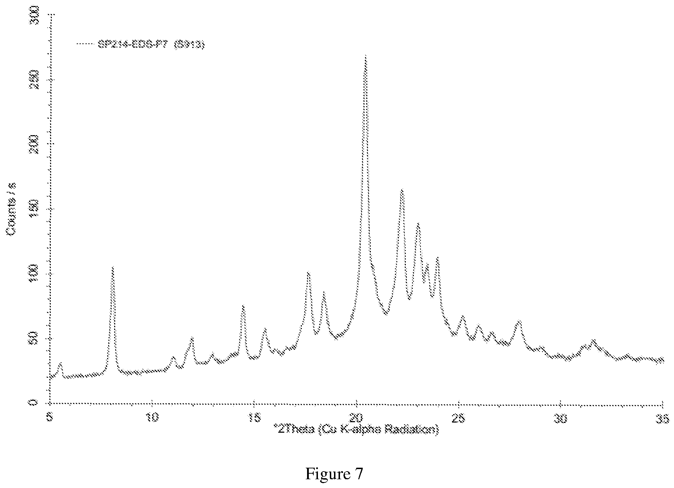

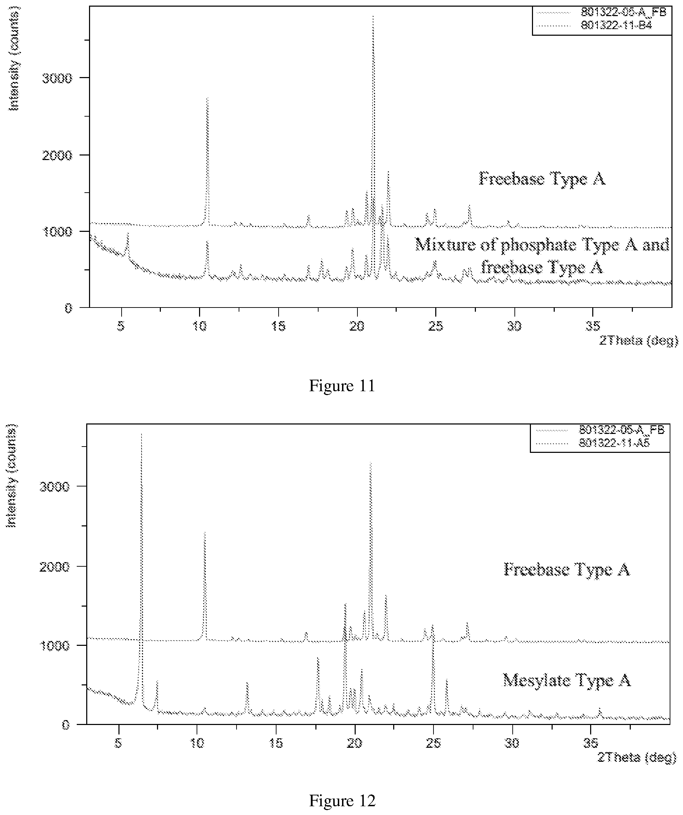

The salt of Compound I can be characterized by an X-ray diffraction pattern substantially similar to that set forth in any one of FIG. 1 to FIG. 12. The salt of Compound I can further be characterized by having a melt onset in a range of about 204 °C to about 207 °C.

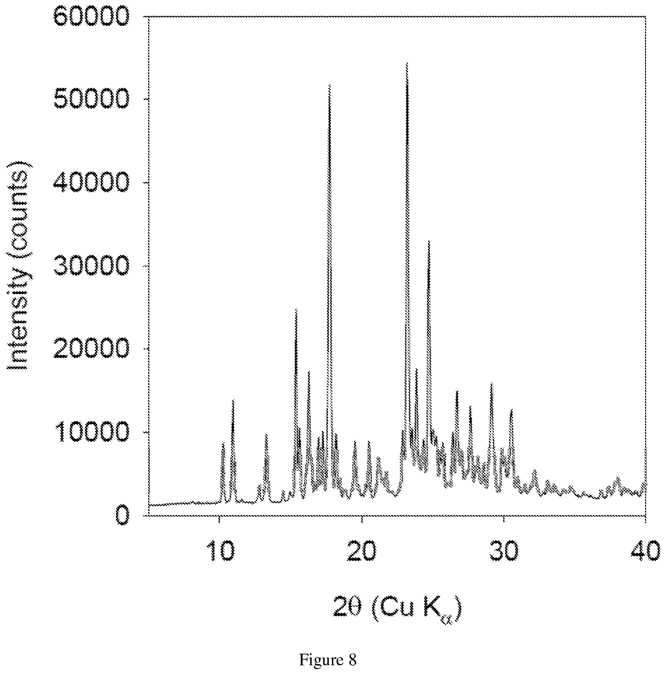

In some embodiments, the salt of Compound I is the mono-tosylate salt. In some embodiments, the mono-tosylate salt is characterized by an X-ray diffraction pattern substantially similar to that set forth in FIG 8. In some embodiments, the mono-tosylate salt is characterized by an X-ray diffraction pattern having three or more peaks selected from those at diffraction angle 2θ values of 10.92°± 0.2°, 13.28°± 0.2°, 15.36°± 0.2°, 16.94°± 0.2°, 17.74°± 0.2°, 18.20°± 0.2°, 20.51°± 0.2°, 23.21°± 0.2°, 23.86°± 0.2°, 24.73°± 0.2°, 25.69°± 0.2°, 26.68°± 0.2°, 27.63°± 0.2°, 29.12°± 0.2°, and 30.532°± 0.2°, when irradiated with a Cu-Kα light source. In some embodiments, the mono-tosylate salt is characterized by an X-ray diffraction pattern having three or more peaks selected from those at diffraction angle 2θ values of 10.92°± 0.2°, 15.36°± 0.2°, 16.94°± 0.2°, 17.74°± 0.2°, 23.21°± 0.2°, 23.86°± 0.2°, 24.73°± 0.2°, 25.69°± 0.2°, 27.63°± 0.2°, and 29.12°± 0.2°, when irradiated with a Cu-Kα light source. In some embodiments, the mono-tosylate salt is characterized by an X-ray diffraction pattern having three or more peaks selected from those at diffraction angle 2θ values of 15.36°± 0.2°, 17.74°± 0.2°, 23.21°± 0.2°, 23.86°± 0.2°, and 24.73°± 0.2°, when irradiated with a Cu-Kα light source.

### Preparation of Compound I and its salts

A synthesis of Compound I and its tosylate salt is shown in the scheme below:

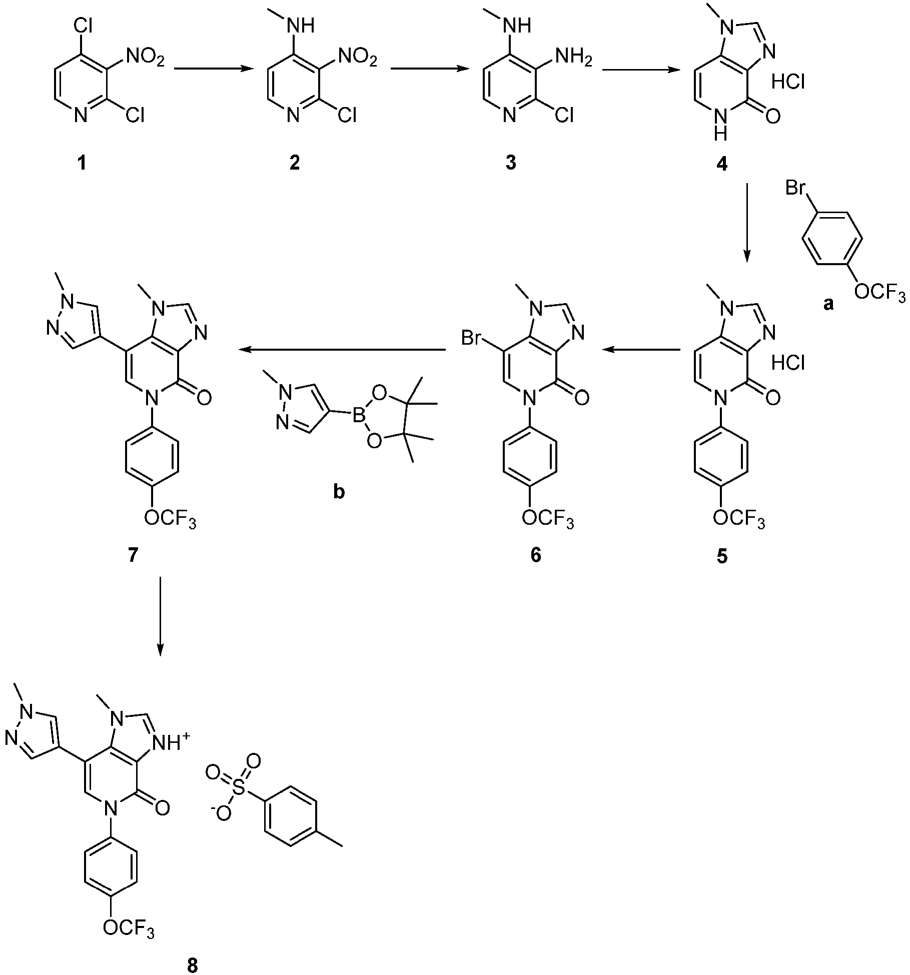

1-methyl-5-(4-(trifluoromethoxy)phenyl)-1,5-dihydro-4H-imidazo[4,5-c]pyridin-4-one (**5**) was synthesized in 4 steps, including a copper-catalyzed coupling reaction *e.g.,* a Goldberg-Ullmann coupling reaction. In another aspect of the invention, intermediate (**5**) is synthesized using any transition metal-catalyzed coupling reaction. The skilled chemist would know that intermediate (**5**) could be synthesized from intermediate (**4**) and compounds of the general formula: 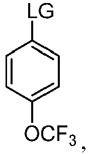 wherein the leaving group "LG" includes but is not limited to halogen, tosylate, mesylate, triflate, etc.

Compound I was synthesized in 6 steps, using a transition metal cross-coupling reaction, *e.g.,* a Suzuki reaction. In another aspect of the invention, Compound I is synthesized using any cross-coupling reaction. Compound I is synthesized from intermediate 6 containing any leaving group. For example, the skilled chemist would use compounds of the general formula: 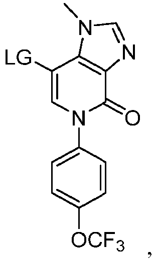 wherein the leaving group "LG" includes but is not limited to halogen, tosylate, mesylate, triflate, etc.

Compound I tosylate was synthesized from the free base form of Compound I via treatment with *p*-toluenesulfonic acid (tosic acid). In one aspect of the invention, Compound I is treated with *p*-toluenesulfonic acid in a solvent. The skilled chemist would contemplate using various appropriate solvents or solvent mixtures to form Compound I tosylate from the free base. In one aspect of the invention, the salt is formed in a solvent mixture, *e.g.*, acetone and water. In another aspect, the salt is formed in methanol. In yet another aspect, the salt is formed in a solvent or solvent mixture which is free of methanol.

In one aspect, the invention relates to a process for preparing Compound I or an intermediate thereof including any one or more of the steps of: (1) reacting 2,4-dichloro-3-nitropyridine with methylamine to yield 2-chloro-N-methyl-3-nitropyridin-4-amine; (2) reducing 2-chloro-N-methyl-3-nitropyridin-4-amine to yield 2-chloro-N⁴-methylpyridine-3,4-diamine; (3) condensing 2-chloro-N⁴-methylpyridine-3,4-diamine with formic acid to yield 1-methyl-1,5-dihydro-4H-imidazo[4,5-c]pyridin-4-one; (4) coupling 1-methyl-1,5-dihydro-4H-imidazo[4,5-c]pyridin-4-one with 1-bromo-4-(trifluoromethoxy)benzene to yield 1-methyl-5-(4-(trifluoromethoxy)phenyl)-1,5-dihydro-4H-imidazo[4,5-c]pyridin-4-one; (5) brominating 1-methyl-5-(4-(trifluoromethoxy)phenyl)-1,5-dihydro-4H-imidazo[4,5-c]pyridin-4-one to yield 7-bromo-1-methyl-5-(4-(trifluoromethoxy)phenyl)-1,5-dihydro-4H-imidazo [4,5-c]pyridin-4-one; and (6) coupling 7-bromo-1-methyl-5-(4-(trifluoromethoxy)phenyl)-1,5-dihydro-4H-imidazo[4,5-c]pyridin-4-one with 1-methyl-4-(4,4,5,5-tetramethyl-1,3,2-dioxaborolan-2-yl)-1H-pyrazole to yield Compound I.

In another aspect, the invention relates to a process for preparing Compound I or an intermediate thereof including any one or more of the steps of: (1) reacting 2,4-dichloro-3-nitropyridine with methylamine to yield 2-chloro-N-methyl-3-nitropyridin-4-amine; (2) reducing 2-chloro-N-methyl-3-nitropyridin-4-amine to yield 2-chloro-N⁴-methylpyridine-3,4-diamine; (3) condensing 2-chloro-N⁴-methylpyridine-3,4-diamine with formic acid to yield 1-methyl-1,5-dihydro-4H-imidazo[4,5-c]pyridin-4-one; (4) coupling 1-methyl-1,5-dihydro-4H-imidazo[4,5-c]pyridin-4-one with 1-bromo-4-(trifluoromethoxy)benzene to yield 1-methyl-5-(4-(trifluoromethoxy)phenyl)-1,5-dihydro-4H-imidazo[4,5-c]pyridin-4-one; (5) brominating 1-methyl-5-(4-(trifluoromethoxy)phenyl)-1,5-dihydro-4H-imidazo[4,5-c]pyridin-4-one to yield 7-bromo-1-methyl-5-(4-(trifluoromethoxy)phenyl)-1,5-dihydro-4H-imidazo [4,5-c]pyridin-4-one; and (6) coupling 7-bromo-1-methyl-5-(4-(trifluoromethoxy)phenyl)-1,5-dihydro-4H-imidazo[4,5-c]pyridin-4-one with 1-methyl-4-(4,4,5,5-tetramethyl-1,3,2-dioxaborolan-2-yl)-1H-pyrazole to yield Compound I, and further including the step of recrystallizing Compound I.

In another aspect, the invention relates to a process for preparing Compound I tosylate or an intermediate thereof including any one or more of the steps of: (1) reacting 2,4-dichloro-3-nitropyridine with methylamine to yield 2-chloro-N-methyl-3-nitropyridin-4-amine; (2) reducing 2-chloro-N-methyl-3-nitropyridin-4-amine to yield 2-chloro-N⁴-methylpyridine-3,4-diamine; (3) condensing 2-chloro-N⁴-methylpyridine-3,4-diamine with formic acid to yield 1-methyl-1,5-dihydro-4H-imidazo[4,5-c]pyridin-4-one; (4) coupling 1-methyl-1,5-dihydro-4H-imidazo[4,5-c]pyridin-4-one with 1-bromo-4-(trifluoromethoxy)benzene to yield 1-methyl-5-(4-(trifluoromethoxy)phenyl)-1,5-dihydro-4H-imidazo[4,5-c]pyridin-4-one; (5) brominating 1-methyl-5-(4-(trifluoromethoxy)phenyl)-1,5-dihydro-4H-imidazo[4,5-c]pyridin-4-one to yield 7-bromo-1-methyl-5-(4-(trifluoromethoxy)phenyl)-1,5-dihydro-4H-imidazo[4,5-c]pyridin-4-one; (6) coupling 7-bromo-1-methyl-5-(4-(trifluoromethoxy)phenyl)-1,5-dihydro-4H-imidazo[4,5-c]pyridin-4-one with 1-methyl-4-(4,4,5,5-tetramethyl-1,3,2-dioxaborolan-2-yl)-1H-pyrazole to yield Compound I; (7) recrystallizing Compound I in a two-solvent system; and (8) contacting Compound I with *p*-toluenesulfonic acid to yield Compound I tosylate.

In another aspect, the invention relates to a process for preparing Compound I tosylate including the step of contacting Compound I with *p*-toluenesulfonic acid to yield Compound I tosylate. In one type of embodiment, the Compound I tosylate is a di-tosylate. In another type of embodiment, the Compound I tosylate is a mono-tosylate.

In another aspect, the invention relates to a process for preparing 7-bromo-1-methyl-5-(4-(trifluoromethoxy)phenyl)-1,5-dihydro-4H-imidazo[4,5-c]pyridin-4-one including the steps of: (1) reacting 2,4-dichloro-3-nitropyridine with methylamine to yield 2-chloro-N-methyl-3-nitropyridin-4-amine; (2) reducing 2-chloro-N-methyl-3-nitropyridin-4-amine to yield 2-chloro-N⁴-methylpyridine-3,4-diamine; (3) condensing 2-chloro-N⁴-methylpyridine-3,4-diamine with formic acid to yield 1-methyl-1,5-dihydro-4H-imidazo[4,5-c]pyridin-4-one; (4) coupling 1-methyl-1,5-dihydro-4H-imidazo[4,5-c]pyridin-4-one with 1-bromo-4-(trifluoromethoxy)benzene to yield 1-methyl-5-(4-(trifluoromethoxy)phenyl)-1,5-dihydro-4H-imidazo[4,5-c]pyridin-4-one; and (5) brominating 1-methyl-5-(4-(trifluoromethoxy)phenyl)-1,5-dihydro-4H-imidazo[4,5-c]pyridin-4-one to yield 7-bromo-1-methyl-5-(4-(trifluoromethoxy)phenyl)-1,5-dihydro-4H-imidazo[4,5-c]pyridin-4-one.

In another aspect, the invention relates to a process for preparing 1-methyl-5-(4-(trifluoromethoxy)phenyl)-1,5-dihydro-4H-imidazo[4,5-c]pyridin-4-one including the steps of: (1) reacting 2,4-dichloro-3-nitropyridine with methylamine to yield 2-chloro-N-methyl-3-nitropyridin-4-amine; (2) reducing 2-chloro-N-methyl-3-nitropyridin-4-amine to yield 2-chloro-N⁴-methylpyridine-3,4-diamine; (3) condensing 2-chloro-N⁴-methylpyridine-3,4-diamine with formic acid to yield 1-methyl-1,5-dihydro-4H-imidazo[4,5-c]pyridin-4-one; and (4) coupling 1-methyl-1,5-dihydro-4H-imidazo[4,5-c]pyridin-4-one with 1-bromo-4-(trifluoromethoxy)benzene to yield 1-methyl-5-(4-(trifluoromethoxy)phenyl)-1,5-dihydro-4H-imidazo[4,5-c]pyridin-4-one.

In another aspect, the invention relates to a process for preparing 1-methyl-1,5-dihydro-4H-imidazo[4,5-c]pyridin-4-one including the steps of: (1) reacting 2,4-dichloro-3-nitropyridine with methylamine to yield 2-chloro-N-methyl-3-nitropyridin-4-amine; (2) reducing 2-chloro-N-methyl-3-nitropyridin-4-amine to yield 2-chloro-N⁴-methylpyridine-3,4-diamine; and (3) condensing 2-chloro-N⁴-methylpyridine-3,4-diamine with formic acid to yield 1-methyl-1,5-dihydro-4H-imidazo[4,5-c]pyridin-4-one.

In another aspect, the invention relates to a process for preparing 2-chloro-N⁴-methylpyridine-3,4-diamine including the steps of: (1) reacting 2,4-dichloro-3-nitropyridine with methylamine to yield 2-chloro-N-methyl-3-nitropyridin-4-amine; and (2) reducing 2-chloro-N-methyl-3-nitropyridin-4-amine to yield 2-chloro-N⁴-methylpyridine-3,4-diamine.

In another aspect, the invention relates to a process for preparing Compound I including the step of: coupling 7-bromo-1-methyl-5-(4-(trifluoromethoxy)phenyl)-1,5-dihydro-4H-imidazo[4,5-c]pyridin-4-one with 1-methyl-4-(4,4,5,5-tetramethyl-1,3,2-dioxaborolan-2-yl)-1H-pyrazole to yield Compound I.

In another aspect, the invention relates to a process for preparing 7-bromo-1-methyl-5-(4-(trifluoromethoxy)phenyl)-1,5-dihydro-4H-imidazo[4,5-c]pyridin-4-one including the step of: brominating 1-methyl-5-(4-(trifluoromethoxy)phenyl)-1,5-dihydro-4H-imidazo[4,5-c]pyridin-4-one to yield 7-bromo-1-methyl-5-(4-(trifluoromethoxy)phenyl)-1,5-dihydro-4H-imidazo[4,5-c]pyridin-4-one.

In another aspect, the invention relates to a process for preparing 1-methyl-5-(4-(trifluoromethoxy)phenyl)-1,5-dihydro-4H-imidazo[4,5-c]pyridin-4-one including the step of: coupling 1-methyl-1,5-dihydro-4H-imidazo[4,5-c]pyridin-4-one with 1-bromo-4-(trifluoromethoxy)benzene to yield 1-methyl-5-(4-(trifluoromethoxy)phenyl)-1,5-dihydro-4H-imidazo[4,5-c]pyridin-4-one.

In another aspect, the invention relates to a process for preparing 1-methyl-1,5-dihydro-4H-imidazo[4,5-c]pyridin-4-one including the step of: condensing 2-chloro-N⁴-methylpyridine-3,4-diamine with formic acid to yield 1-methyl-1,5-dihydro-4H-imidazo[4,5-c]pyridin-4-one.

In another aspect, the invention relates to a process for preparing 2-chloro-N⁴-methylpyridine-3,4-diamine including the step of: reducing 2-chloro-N-methyl-3-nitropyridin-4-amine to yield 2-chloro-N⁴-methylpyridine-3,4-diamine.

In another aspect, the invention relates to a process for preparing 2-chloro-N-methyl-3-nitropyridin-4-amine including the step of: reacting 2,4-dichloro-3-nitropyridine with methylamine to yield 2-chloro-N-methyl-3-nitropyridin-4-amine.

An alternative synthesis of Compound I and its salts is shown in the scheme below:

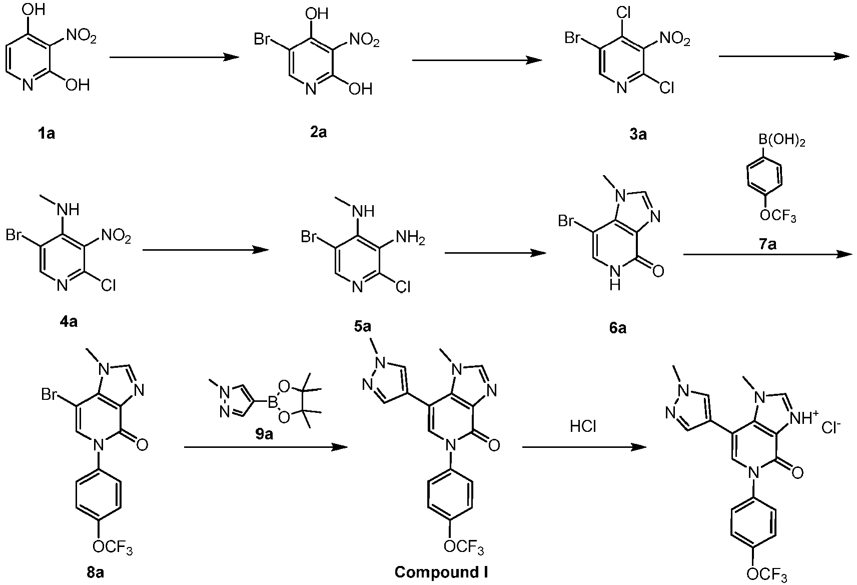

In this synthesis, intermediate (**2a**) is produced from 3-nitropyridine-2,4-diol (**1a**) by treating 3-nitropyridine-2,4-diol (**1a**) with a brominating agent. The brominating agent may be selected from any suitable brominating agent, including those conventionally known in the art, such as molecular bromine, N-bromosuccinimide, etc.

Intermediate (**3a**) is produced from intermediate (**2a**) by treating intermediate (**3a**) with a chlorinating agent. The chlorinating agent may be selected from any suitable chlorinating agent, including those conventionally known in the art, such as molecular chlorine, N-chlorosuccinimide, phosphoryl chloride and derivatives thereof, etc.

Intermediate (**8a**) is produced from 3-nitropyridine-2,4-diol (**1a**) in 6 steps. Intermediate (**8a**) is produced via a transition metal-catalyzed coupling reaction *e.g.,* a Chan-Lam coupling reaction. In another aspect of the invention, intermediate (**8a**) is synthesized using any transition metal-catalyzed coupling reaction.

Compound I can be synthesized in 7 steps, using a transition metal cross-coupling reaction, *e.g.,* a Suzuki reaction. In another aspect of the invention, Compound I can be synthesized using any cross-coupling reaction. Compound I can be synthesized from intermediate (**8a**) containing any leaving group. For example, the skilled chemist can use compounds of the general formula: 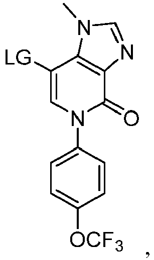 wherein the leaving group "LG" includes but is not limited to halogen, tosylate, mesylate, triflate, etc.

Any process for making Compound I can optionally include a step of recrystallizing Compound I, further optionally in solvent system having at least two solvents or consisting of two solvents. For example, the solvent system can include or consist of acetic acid and ethanol. One of the solvents in a two solvent system can be present in volumetric excess. For example, in a solvent system including or consisting of acetic acid and ethanol, the ethanol can be present in volumetric excess compared to acetic acid. The solvent ratios may be varied as needed to achieve a desired purity and/or yield in the recrystallization. For example, a solvent system can contain acetic acid and ethanol in a v/v ratio of about 1:1 to about 1:15, about 1:1 to about 1:10, about 1:4 to about 1:10, or about 1:6 to about 1:8 acetic acid: ethanol.

Compound I hydrochloride can be synthesized from the free base form of Compound I via treatment with hydrochloric acid. In one aspect of the invention, Compound I is treated with hydrochloric acid in a solvent. The skilled chemist would contemplate using various appropriate solvents or solvent mixtures to form Compound I hydrochloride from the free base. In one aspect of the invention, the salt is formed in a solvent mixture, e.g., dioxane and water. In another aspect, the salt is formed in methanol. In yet another aspect, the salt is formed in a solvent or solvent mixture which is free of methanol.

A salt of Compound I can be prepared, e.g. by milling, to a desired particle size. For example, the particle size can range from nanometer scale to low micrometer scale, e.g. 500 µm or less, or 100 µm or less volume mean diameter. In one type of embodiment, the majority of the particles will be in a range of 1 µm to 100 µm, or 1 µm to 10 µm. Particle size distributions can be characterized by D10, D50, D90, and D[4,3] values, as is known in the art. In one type of embodiment, the Compound I salt (e.g. tosylate salt) particles can have a D50 in a range of about 10 µm to about 60 µm, or about 10 µm to about 35 µm, or about 25 µm to about 30 µm. The Compound I salt (e.g. tosylate salt) particles can have a volume mean diameter D[4,3] in a range of about 10 µm to about 60 µm, or about 25 µm to about 45 µm, or about 30 µm to about 40 µm. The Compound I salt (e.g. tosylate salt) particles can have a D90 in a range of about 50 µm to about 100 µm, or about 60 µm to about 90 µm. The Compound I salt (e.g. tosylate salt) particles can have a D10 in a range of about 1 µm to about 20 µm, or about 1 µm to about 15 µm, or about 5 µm to about 10 µm.

A salt of Compound I can be prepared into a pharmaceutical composition, e.g. by addition of one or more excipients and through compounding or other processing steps. Standard pharmaceutical formulation techniques can be used, such as those disclosed in Remington's The Science and Practice of Pharmacy, 21st Ed., Lippincott Williams & Wilkins (2005), incorporated by reference in its entirety.

In addition to the Compound I salt, embodiments include compositions containing a pharmaceutically-acceptable carrier or other excipient. The term "pharmaceutically-acceptable carrier", as used herein, means one or more compatible solid or liquid filler diluents or encapsulating substances, which are suitable for administration to a mammal. The term "compatible", as used herein, means that the components of the composition are capable of being commingled with the subject Compound I salt, and with each other, in a manner such that there is no interaction that would substantially reduce the pharmaceutical efficacy of the composition under ordinary use situations. Pharmaceutically-acceptable carriers must, of course, be of sufficiently high purity and sufficiently low toxicity to render them suitable for administration preferably to an animal, preferably mammal being treated. Suitable excipients are described in the Handbook of Pharmaceutical Excipients (Rowe, Ed., APhA Publications, 2017). Excipients can be intragranular (i.e., incorporated into the granule) or extragranular (i.e., outside of the granule).

Some examples of substances which can serve as pharmaceutically-acceptable excipients, carriers, or components thereof, are dicalcium phosphate, calcium sulfate, sugars, such as lactose, glucose and sucrose; starches, such as corn starch and potato starch; cellulose and its derivatives, such as sodium carboxymethyl cellulose, ethyl cellulose, and methyl cellulose; kaolin, powdered tragacanth; malt; gelatin; talc; solid lubricants, such as stearic acid, magnesium stearate, and calcium stearate; calcium sulfate; mineral oil; vegetable oils, such as hydrogenated vegetable oil, peanut oil, cottonseed oil, sesame oil, olive oil, corn oil and oil of theobroma; polyols such as propylene glycol, glycerine, sorbitol, inositol, mannitol, and polyethylene glycol; alginic acid; emulsifiers, such as the TWEENS; wetting agents, such sodium lauryl sulfate; coloring agents; flavoring agents; tableting agents, stabilizers; antioxidants; preservatives; pyrogen-free water; isotonic saline; and phosphate buffer solutions.

The choice of a pharmaceutically-acceptable carrier to be used in conjunction with the subject compound is basically determined by the way the compound is to be administered.

The Compound I salts described herein can be provided in unit dosage form. As used herein, a "unit dosage form" is a composition containing an amount of a compound that is suitable for administration to an animal, preferably mammal subject, in a single dose, according to good medical practice. The preparation of a single or unit dosage form however, does not imply that the dosage form is administered once per day or once per course of therapy. Such dosage forms are contemplated to be administered once, twice, thrice or more per day and may be administered as infusion over a period of time (e.g., from about 30 minutes to about 2-6 hours), or administered as a continuous infusion, and may be given more than once during a course of therapy, though a single administration is not specifically excluded. The skilled artisan will recognize that the formulation does not specifically contemplate the entire course of therapy and such decisions are left for those skilled in the art of treatment rather than formulation.

The compositions useful as described above may be in any of a variety of suitable forms for a variety of routes for administration, for example, for oral, nasal, rectal, topical (including transdermal), ocular, intracerebral, intracranial, intrathecal, intra-arterial, intravenous, intramuscular, or other parental routes of administration. The skilled artisan will appreciate that oral and nasal compositions include compositions that are administered by inhalation, and made using available methodologies. Depending upon the particular route of administration desired, a variety of pharmaceutically-acceptable carriers well-known in the art may be used. Pharmaceutically-acceptable carriers include, for example, solid or liquid fillers, diluents, hydrotropies, surface-active agents, and encapsulating substances. Optional pharmaceutically-active materials that do not substantially interfere with the inhibitory activity of the compound may be included. The amount of carrier employed in conjunction with the compound is sufficient to provide a practical quantity of material for administration per unit dose of the compound. Techniques and compositions for making dosage forms useful in the methods described herein are described in the following references, all incorporated by reference herein: Modern Pharmaceutics, 4th Ed., Chapters 9 and 10 (Banker & Rhodes, editors, 2002); Lieberman et al., Pharmaceutical Dosage Forms: Tablets (1989); and Ansel, Introduction to Pharmaceutical Dosage Forms 8th Edition (2004).

Various oral dosage forms can be used, including such solid forms as pills, tablets, cores, capsules, caplets, granules, suspensions, nanosuspensions, and bulk powders. Tablets can be compressed, tablet triturates, enteric-coated, sugar-coated, film-coated, or multiple-compressed, containing suitable binders, fillers, lubricants, diluents, disintegrating agents, coloring agents, flavoring agents, flow-inducing agents, and melting agents. Liquid oral dosage forms include aqueous solutions, emulsions, suspensions, solutions and/or suspensions reconstituted from non-effervescent granules, and effervescent preparations reconstituted from effervescent granules, containing suitable solvents, preservatives, emulsifying agents, suspending agents, diluents, sweeteners, melting agents, coloring agents and flavoring agents, or combinations of any of these.

The pharmaceutically-acceptable carriers suitable for the preparation of unit dosage forms for peroral administration is well-known in the art. Tablets typically include one or more conventional pharmaceutically-compatible adjuvants as inert diluents or fillers, such as inorganic salts (e.g., calcium carbonate, calcium phosphate, calcium sulfate, sodium carbonate), sugar alcohols (e.g., xylitol, sorbitol, mannitol, maltitol), sugars (e.g. sucrose, glucose, dextrose, molasses, lactose) and cellulose, or any combination thereof; binders such as starch, gelatin, sugars (e.g. sucrose, glucose, dextrose, molasses, lactose), sugar alcohols (e.g., xylitol, sorbitol, mannitol, maltitol), natural and synthetic gums (acacia, alginic acid, sodium alginate, extract of Irish moss, panwar gum, ghatti gum, cellulose, microcrystalline cellulose, carboxymethylcellulose, hydroxypropyl methylcellulose (HPMC or hypromellose), methyl cellulose, ethyl cellulose, hydroxypropyl cellulose, sodium carboxymethyl cellulose (croscarmellose sodium), polyvinylpyrrolidone (PVP), Veegum^{®} (magnesium aluminum silicate), and arabogalactan), proteins, and polymers, hydroxypropyl methylcellulose (hypromellose), tragacanth, polyvinyl alcohols, polymethacrylates , and polyethylene glycol (PEG), or any combination thereof; disintegrants such as a sugar, starch, a modified starch, alginic acid, a crosslinked polymer, sodium starch glycolate, polyvinylpovidone, and croscarmellose, or combinations thereof; lubricants such as calcium stearate, magnesium stearate, stearic acid, glyceryl behenate, mineral oil, hydrogenated vegetable oils, polyethylene glycol, sodium lauryl sulfate, glyceryl palmitostearate, sodium benzoate, sodium stearyl fumarate, silica, and talc. Glidants such as silicon dioxide can be used to improve flow characteristics of the powder mixture. Coloring agents, such as the FD&C dyes, can be added for appearance. Sweeteners and flavoring agents, such as aspartame, saccharin, menthol, peppermint, and fruit flavors, are useful adjuvants for chewable tablets. Capsules typically comprise one or more solid diluents disclosed above. The selection of carrier components depends on secondary considerations like taste, cost, and shelf stability, which are not critical, and can be readily made by a person skilled in the art.

Peroral compositions also include liquid solutions, emulsions, suspensions, and the like. The pharmaceutically-acceptable carriers suitable for preparation of such compositions are well known in the art. Typical components of carriers for syrups, elixirs, emulsions and suspensions include ethanol, glycerol, propylene glycol, polyethylene glycol, liquid sucrose, sorbitol and water. For a suspension, typical suspending agents include methyl cellulose, sodium carboxymethyl cellulose, AVICEL RC-591, tragacanth and sodium alginate; typical wetting agents include lecithin and polysorbate 80; and typical preservatives include methyl paraben and sodium benzoate. Peroral liquid compositions may also contain one or more components such as sweeteners, flavoring agents and colorants disclosed above.

Such compositions may also be coated by conventional methods, typically with pH or time-dependent coatings, such that the subject compound is released in the gastrointestinal tract in the vicinity of the desired topical application, or at various times to extend the desired action. Such dosage forms typically include, but are not limited to, one or more of cellulose acetate phthalate, polyvinylacetate phthalate, hydroxypropyl methyl cellulose phthalate, ethyl cellulose, Eudragit^{®} (copolymers derived from esters of acrylic and methacrylic acid) coatings, waxes and shellac.

Compositions described herein may optionally include additional, secondary drug actives different from the Compound I salts.

Other compositions useful for attaining systemic delivery of the subject compounds include sublingual, buccal and nasal dosage forms. Such compositions typically comprise one or more of soluble filler substances such as sucrose, sorbitol and mannitol; and binders such as acacia, microcrystalline cellulose, carboxymethyl cellulose and hydroxypropyl methyl cellulose. Glidants, lubricants, sweeteners, colorants, antioxidants and flavoring agents disclosed above may also be included.

A liquid composition may be formulated such that it can be administered topically to the eye. The comfort should be maximized as much as possible, although sometimes formulation considerations (e.g. drug stability) may necessitate less than optimal comfort. In the case that comfort cannot be maximized, the liquid should be formulated such that the liquid is tolerable to the patient for topical ophthalmic use. Additionally, an ophthalmically acceptable liquid should either be packaged for single use, or contain a preservative to prevent contamination over multiple uses.

For ophthalmic application, solutions or medicaments are often prepared using a physiological saline solution as a major vehicle. Ophthalmic solutions should preferably be maintained at a comfortable pH with an appropriate buffer system. The formulations may also contain conventional, pharmaceutically acceptable preservatives, stabilizers and surfactants.

Preservatives that may be used in the pharmaceutical compositions disclosed herein include, but are not limited to, benzalkonium chloride, PHMB, chlorobutanol, thimerosal, phenylmercuric acetate and phenylmercuric nitrate. A useful surfactant is, for example, Tween^{®} 80 (polyoxyethylene (20) sorbitan monooleate). Likewise, various useful vehicles may be used in the ophthalmic preparations disclosed herein. These vehicles include, but are not limited to, polyvinyl alcohol, povidone, hydroxypropyl methyl cellulose, poloxamers, carboxymethyl cellulose, hydroxyethyl cellulose and purified water.

Tonicity adjustors may be added as needed or convenient. They include, but are not limited to, salts, particularly sodium chloride, potassium chloride, mannitol and glycerin, or any other suitable ophthalmically acceptable tonicity adjustor.

Various buffers and means for adjusting pH may be used so long as the resulting preparation is ophthalmically acceptable. For many compositions, the pH will be between 4 and 9. Accordingly, buffers include acetate buffers, citrate buffers, phosphate buffers and borate buffers. Acids or bases may be used to adjust the pH of these formulations as needed.

In a similar vein, an ophthalmically acceptable antioxidant includes, but is not limited to, sodium metabisulfite, sodium thiosulfate, acetylcysteine, butylated hydroxyanisole and butylated hydroxy toluene.

Other excipient components that may be included in the ophthalmic preparations are chelating agents. A useful chelating agent is edetate disodium, although other chelating agents may also be used in place or in conjunction with it.

For topical use, creams, ointments, gels, solutions or suspensions, etc., containing a Compound I salt disclosed herein are employed. Topical formulations may generally be comprised of a pharmaceutical carrier, co-solvent, emulsifier, penetration enhancer, preservative system, and emollient.

For intravenous administration, the compounds and compositions described herein may be dissolved or dispersed in a pharmaceutically acceptable diluent, such as a saline or dextrose solution. Suitable excipients may be included to achieve the desired pH, including but not limited to NaOH, sodium carbonate, sodium acetate, HCl, and citric acid. In various embodiments, the pH of the final composition ranges from 2 to 8, or preferably from 4 to 7. Antioxidant excipients may include sodium bisulfite, acetone sodium bisulfite, sodium formaldehyde, sulfoxylate, thiourea, and EDTA. Other non-limiting examples of suitable excipients found in the final intravenous composition may include sodium or potassium phosphates, citric acid, tartaric acid, gelatin, and carbohydrates such as dextrose, mannitol, and dextran. Further acceptable excipients are described in Powell, et al., Compendium of Excipients for Parenteral Formulations, PDA J Pharm Sci and Tech 1998, 52 238-311 and Nema et al., Excipients and Their Role in Approved Injectable Products: Current Usage and Future Directions, PDA J Pharm Sci and Tech 2011, 65 287-332, both of which are incorporated herein by reference in their entirety. Antimicrobial agents may also be included to achieve a bacteriostatic or fungistatic solution, including but not limited to phenylmercuric nitrate, thimerosal, benzethonium chloride, benzalkonium chloride, phenol, cresol, and chlorobutanol.

The compositions for intravenous administration may be provided to caregivers in the form of one more solids that are reconstituted with a suitable diluent such as sterile water, saline or dextrose in water shortly prior to administration. In other embodiments, the compositions are provided in solution ready to administer parenterally. In still other embodiments, the compositions are provided in a solution that is further diluted prior to administration. In embodiments that include administering a combination of a compound described herein and another agent, the combination may be provided to caregivers as a mixture, or the caregivers may mix the two agents prior to administration, or the two agents may be administered separately.

The actual dose of the active compounds described herein depends on the specific compound, and on the condition to be treated; the selection of the appropriate dose is well within the knowledge of the skilled artisan.

In any embodiment of the methods or kits described herein, the effective daily amount of a Compound I salt can be from about 1 mg to about 5000 mg per day, about 5 mg to about 2500 mg per day, or about 10 mg to about 2000 mg per day, on a free base basis. In some further embodiments, the amount of a Compound I salt administered is from about 25 mg to about 1600 mg per day, on a free base basis. In some further embodiments, the amount of a Compound I salt administered is about 25 mg, about 75 mg, about 200 mg, about 275 mg, about 400 mg, about 550 mg, about 575 mg, about 800 mg, about 1150 mg, or about 1600 mg per day, on a free base basis, or in a range defined by any two of the preceding values.

Solid compositions, including solid dosage forms and oral solid dosage forms, are particularly contemplated, e.g. tablets and capsules. The compositions can be made of or include a granulate including a Compound I salt, e.g. by wet granulation, fluid bed granulation, compression granulation, or extrusion spheronization. In view of the hygroscopic nature of Compound I salts, dry granulation processes, and dry-granulated compositions are particularly contemplated. Examples include anhydrous wet granulation (e.g. using ethanol as a binder solvent), anhydrous fluid bed granulation, and compression granulation (e.g., by use of slugging or roller compaction). Tablets can also be made by direct compression.

The pharmaceutical composition, granulation, or dosage form can suitably include a polymeric precipitation inhibitor. Polymeric precipitation inhibitors include cellulose acetate phthalate, carbomers, ethyl cellulose, Eudragit^{®}, alginic acid, gum Arabic, locust bean xanthan, hydroxyethyl cellulose, hydroxypropyl cellulose, hydroxypropyl methylcellulose (e.g. Methocel^{®} K15M), hydroxypropyl methylcellulose acetate succinate, hydroxypropyl methylcellulose phthalate, methyl cellulose, methyl 2-hydroxyethyl cellulose, poly(acrylic acid), polyallylamine hydrogen chloride, poly(acrylamide-co-acrylic acid), polydiallyldimethyl ammonium chloride, polyethylene imine, P-EPE, poly(2-ethyl 2-oxazoline), polyvinyl alcohol, polyvinyl pyrrolidone, sodium carboxymethyl cellulose, or any combination thereof. Hydrophobic polymers are contemplated. Cellulosics are particularly contemplated, for example cellulose acetate phthalate, ethyl cellulose, hydroxyethyl cellulose, hydroxypropyl cellulose, hydroxypropyl methylcellulose, hydroxypropyl methylcellulose acetate succinate, hydroxypropyl methylcellulose phthalate, methyl cellulose, methyl 2-hydroxyethyl cellulose. A hydroxypropyl methylcellulose having a 2% solution viscosity in water at 20 °C in a range of about 13,000 to about 25,000 mPa·s is preferred, e.g. Methocel^{®} K15M. The polymeric precipitation inhibitor (e.g. HPMC) can be in an outer region of the dosage form, e.g. in an outer layer, an outer coating, or as an extragranular component. The polymeric precipitation inhibitor (e.g. HPMC) is present in an amount effective to inhibit precipitation of the Compound I, compared to a composition omitting the polymeric precipitation inhibitor. For example, the polymeric precipitation inhibitor (e.g. HPMC) can be present in an amount of at least 0.1 wt.% in the dosage form, or at least 0.2 wt.%, or at least 0.5 wt.%, and can be present in an amount up to 50 wt.%, or 40 wt.%, or 30 wt.%, or 20 wt.%, or 10 wt.%, or 5 wt.%, or 3 wt.%, for example in a range of 0.5 wt.% to 5 wt.%. In some embodiments, the polymeric precipitation inhibitor (e.g. HPMC) is present in an amount of about 0.5 wt.% in the dosage form.

Granules including a Compound I salt (e.g. tosylate salt) can include a binder (e.g. microcrystalline cellulose), filler (e.g. lactose), and disintegrant (e.g. croscarmellose sodium), for example. The granulate can further include a lubricant (e.g. magnesium stearate). A tablet or other dosage form including the granules can further include extragranular polymeric precipitation inhibitor (e.g. hydroxypropyl methylcellulose). The extragranular portion of the dosage form can include a binder (e.g. microcrystalline cellulose), for example. The extragranular portion of the dosage form can also include disintegrant (e.g. croscarmellose sodium), for example. The extragranular portion of the dosage form can further include a lubricant (e.g. magnesium stearate). The extragranular portion of the dosage form can include combinations of excipients, such as a disintegrant, a lubricant, a binder, or any combination thereof. For example, the extragranular portion of the dosage form can include a disintegrant and a binder, a lubricant, or a polymeric precipitation inhibitor.

The oral dosage form can have any suitable strength, e.g. in a range of 1 mg to 2000 mg Compound I salt (e.g. tosylate), or 10 mg to 1000 mg, or 25 mg to 400 mg, or 100 mg to 300 mg, for example. In another type of embodiment, the oral dosage from can have a strength defined on the Compound I free base basis, e.g. in a range of 1 mg to 2000 mg Compound I free base, or 10 mg to 1000 mg, or 25 mg to 400 mg, or 100 mg to 300 mg, for example.

The pharmaceutical composition (e.g., granules, tablets, capsules) can include granulates having various proportions of Compound I salt (e.g., tosylate) and excipients. For example, a granulate can have about 30 wt% to about 40 wt% Compound I tosylate, about 40 wt% to about 45 wt% binder, about 10 wt% to about 20 wt% filler, and about 0.5 wt% to about 5 wt% disintegrant. The granulate can further include about 0.5% to about 5% lubricant.

The granulate can include e.g., a salt of Compound I, microcrystalline cellulose, lactose, sodium carboxymethyl cellulose, and magnesium stearate. The granulate can further include one or both of extragranular sodium starch glycolate and hydroxypropyl methylcellulose. The granulate can further include extragranular magnesium stearate.

The pharmaceutical composition (e.g., granules, tablets, capsules) can include any salt of Compound I. In one type of embodiment, the salt is mono-HBr amorphous, mono-HBr crystalline, di-HBr crystalline, mono-HCl amorphous, mono-HCl crystalline, di-HCl crystalline, mono-nitrate amorphous, mono-nitrate crystalline, mono-sulfate amorphous, mono-sulfate crystalline, besylate, hemi-edisylate, hemi-napadisylate, mono-tosylate, or di-tosylate. In another type of embodiment, the salt is mono-HBr amorphous, mono-HBr crystalline, di-HBr crystalline, di-HCl crystalline, mono-nitrate amorphous, mono-nitrate crystalline, mono-sulfate amorphous, mono-sulfate crystalline, besylate, hemi-edisylate, hemi-napadisylate, mono-tosylate, or di-tosylate. In another type of embodiment, the salt is mono-HBr amorphous, mono-HBr crystalline, di-HBr crystalline, mono-nitrate amorphous, mono-nitrate crystalline, mono-sulfate amorphous, mono-sulfate crystalline, besylate, hemi-edisylate, hemi-napadisylate, mono-tosylate, or di-tosylate. In another type of embodiment, the salt is sulfate, di-hydrobromide, nitrate, besylate, hemi-edisylate, hemi-napadisylate, mono-tosylate, or di-tosylate. In another type of embodiment, the salt is mono-tosylate, or di-tosylate. In another type of embodiment, the salt is not an HCl salt.

The pharmaceutical composition can also be a suspension, e.g. a nanosuspension, including Compound I free base. The particle size of Compound I free base can be on any desired scale, e.g. the nanometer scale, e.g. in a range of 1 nm to 1000nm. In some embodiments Compound I free base has a particle size (D90) between 1 nm and 1000 nm, between 1 nm and 900 nm, between 1 nm and 800 nm, between 1 nm and 700 nm, between 1 nm and 600 nm, between 1 nm and 500 nm, between 1 nm and 400 nm, between 1 nm and 300 nm, between 1 nm and 200 nm, between 1 nm and 100 nm, between 1 nm and 50 nm, or between 1 nm and 10 nm, as measured by a dynamic light scattering or laser diffraction analyzer. The following description is provided for a nanosuspension, while other particle sizes could also be used in the formulation.

The nanosuspension can include a vehicle. Water is a suitable vehicle. The water can be sterilized, and optionally deionized.

The nanosuspension can include a polymer additive to stabilize the suspension. Suitable polymers include, for example povidone(PVP), methylcellulose, hydroxypropyl cellulose, and HPMC. A polymer may also function to inhibit crystal growth. Other suspension stabilizers include gums, sorbitol, glycerol, polyvinyl alcohol, polyethylene oxide, and other cellulose derivatives, including other alkyl ethers of cellulose, such as ethylcellulose, hydroxyethylcellulose, hydroxyethylmethylcellose and combinations thereof.

The nanosuspension can include a surfactant. A surfactant can contribute to the improvement in the suspension stability by modifying surface tension, which also allows for reduction in interfacial tension between the continuous phase and the dispersion of the suspension. Surfactants can be selected from anionic, nonionic, cationic, and amphoteric. In one type of embodiment, the surfactant will include a non-ionic surfactant. For example, a non-ionic surfactant can be selected from one or more ethoxylates (e.g., fatty alcohol ethoxylates, alkylphenol ethoxylates, fatty acid ethoxylates) and fatty acid esters of polyhydroxy compounds (e.g., fatty acid esters of glycerol, fatty acid esters of sorbitol), including sorbitan esters, ethoxylated sorbitan and fatty acids, polyoxyethylene alkyl-phenols, polyoxyethylene alcohols, polyoxyethylene esters of fatty acids, polyoxyethylene mercaptans polyoxyethylene alkyl amines, and nonyl-phenoxy-polyethoxyethanol. In another type of embodiment, the surfactant is anionic. For example, the anionic surfactant can include one or more of sodium dodecyl sulfate (SDS, a.k.a. sodium lauryl sulfate), dicetyl phosphate, aerosol-OT, and hydroxyprophylmethylcellulose acetate succinate. It was found that in a nanosuspension including polysorbate 80, there was chemical instability. Without intending to be bound by any particular theory, it is believed that the instability was due to an oxidative mechanism. Accordingly, preferably the surfactant will be one having a low or no peroxide content (e.g., ultra-purity polysorbate 80), or an alternative surfactant having no peroxide content, e.g. SDS.

In one type of embodiment, the nanosuspension includes both a polymer stabilizer and a surfactant. For example, the nanosuspension can include a non-ionic polymer, e.g. HPMC, and an anionic surfactant, e.g. SDS.

When a surfactant is added to the composition it is present in an amount in a range of about 0.1 wt.% to about 4.0 wt.% or about 0.5 wt.%.to about 2.0 wt.%.

The nanosuspension can further include other optional excipients, including one or more of pH adjusting or regulating agents (e.g. buffers), a preservative agent to avoid the growth of microorganisms, taste-masking agents, sweeteners, and flavorings.

Agents suitable for regulating the pH of the nanosuspension can include, for example, potassium acetate, sodium acetate, acetic acid, adipic acid, boric acid, citric acid, hydrochloric acid, fumaric acid, malic acid, nitric acid, propionic acid, succinic acid, sulfuric acid, tartaric acid, potassium bicarbonate, sodium bicarbonate, ammonium carbonate, sodium carbonate, potassium citrate, sodium citrate, diethanolamine, ammonium phosphate, potassium phosphate, sodium phosphate, sodium glycolate, ammonium hydroxide, sodium hydroxide, sodium lactate or sodium propionate, among others, or their mixtures.

Preservatives suitable for pharmaceutical compositions, e.g. those for oral use, are well known in the field and can be chosen from, for example, benzoic acid, sodium benzoate, potassium benzoate, parabens such as methylparaben, ethylparaben, propylparaben and butylparaben, sorbic acid, and potassium sorbate, among others, or their mixtures. In one type of embodiment, the preservative is selected from sodium benzoate, methylparaben, ethylparaben, propylparaben, or a combination thereof.

It is contemplated to prepare a dosage formulation, optionally an oral dosage formulation, e.g. a solid oral dosage formulation, e.g. a granulate and/or a tablet, by including such a nanosuspension in the formulation. Such an oral dosage formulation can be made by a process that includes drying the nanosuspension. In one type of embodiment, the process will include:(a) granulating the nanosuspension to produce a granulated nanosuspension; (b) adding mannitol to the granulated nanosuspension prepared in step (a) to produce a nanosuspension mixture; (c) spraying the nanosuspension mixture prepared in step (b) onto a fluid bed charged with microcrystalline cellulose to produce a nanosuspension wet blend; (d) raising the temperature of the fluid bed to above 40 °C to dry the nanosuspension wet blend to produce a nanosuspension dry blend; and (e) milling the nanosuspension dry blend to produce nanosuspension granules. The nanosuspension granules can optionally be pressed into a dosage form (e.g., pill or tablet). The granules can have any desired particle size, e.g. a particle size D₅₀ in a range of about 100 µm to about 170 µm, or about 130 µm to about 140 µm, or about 135 µm.

A pharmaceutical formulation described herein can be made by any suitable process, including those conventionally known in the art. The process can include a size-modification step, e.g. milling, to yield a desired particle size of the Compound I or salt or solvate thereof, e.g. in the size ranges described herein. The process can also include a size selection step, e.g. sieving, to yield a desired particle size range or threshold. One or more excipients can also be sieved to a desired particle size range or threshold. The dry components can be blended together, and with a lubricant. The mixture of active and excipients can be compressed. Optionally, the compressed mixture can be ground to yield granules of a desired particle size. Extragranular excipients can be added to and mixed with the ground mixture. Optionally, an extragranular lubricant can also be used. The resulting mixture of granules and extragranular excipients can be compressed into a tablet. For example, one process includes (a) sieving a Compound I salt, a binder, a filler, and a disintegrant; (b) blending the sieved components to form a first mixture; (c) further blending said first mixture with a lubricant to form a second mixture; (d) compressing the second mixture; (e) grinding the compressed second mixture; (e) blending the ground second mixture with an extragranular disintegrant and an extragranular binder to form a third mixture; (f) blending said third mixture with an extragranular lubricant to form a fourth mixture; and (g) compressing said fourth mixture to form a tablet. In any such process, the active can be a Compound I tosylate salt, e.g. the mono-tosylate salt.

Also disclosed are methods of treating a fibrotic condition, including administering a therapeutically effective amount of a Compound I salt (e.g. tosylate), or a pharmaceutical composition thereof as described herein. In some such embodiments, the method further comprises identifying the subject as having or at risk of having said fibrotic condition. In some such embodiments, the fibrotic condition is selected from the group consisting of pulmonary fibrosis, dermal fibrosis, pancreatic fibrosis, liver fibrosis, and renal fibrosis. In some embodiment, the fibrotic condition is idiopathic pulmonary fibrosis. In some embodiments, the subject receiving such method of treatment is a human.

"Treat," "treatment," or "treating," as used herein refers to administering a compound or pharmaceutical composition to a subject for prophylactic and/or therapeutic purposes. The term "prophylactic treatment" refers to treating a subject who does not yet exhibit symptoms of a disease or condition, but who is susceptible to, or otherwise at risk of, a particular disease or condition, whereby the treatment reduces the likelihood that the patient will develop the disease or condition. The term "therapeutic treatment" refers to administering treatment to a subject already suffering from a disease or condition.

A "fibrotic condition," "fibroproliferative condition," "fibrotic disease," "fibroproliferative disease," "fibrotic disorder," and "fibroproliferative disorder" are used interchangeably to refer to a condition, disease or disorder that is characterized by dysregulated proliferation or activity of fibroblasts and/or abnormal accumulation of fibronectin and/or pathologic or excessive accumulation of collagenous tissue. Typically, any such disease, disorder or condition is amenable to treatment by administration of a compound having anti-fibrotic activity. Fibrotic disorders include, but are not limited to, pulmonary fibrosis, including idiopathic pulmonary fibrosis (IPF) and pulmonary fibrosis from a known etiology, dermal fibrosis, pancreatic fibrosis, liver fibrosis (e.g., hepatic fibrosis associated with chronic active hepatitis), and renal fibrosis.

In other embodiments of the present disclosure the disease or condition to be treated can include pulmonary fibrosis, idiopathic pulmonary fibrosis, idiopathic interstitial pneumonia, autoimmune lung diseases, benign prostate hypertrophy, coronary or myocardial infarction, atrial fibrillation, cerebral infarction, myocardiac fibrosis, musculoskeletal fibrosis, post-surgical adhesions, liver cirrhosis, renal fibrotic disease, fibrotic vascular disease, scleroderma, Hermansky-Pudlak syndrome, neurofibromatosis, Alzheimer's disease, diabetic retinopathy, and/or skin lesions, lymph node fibrosis associated with HIV, chronic obstructive pulmonary disease (COPD), inflammatory pulmonary fibrosis, rheumatoid arthritis; rheumatoid spondylitis; osteoarthritis; gout, other arthritic conditions; sepsis; septic shock; endotoxic shock; gram-negative sepsis; toxic shock syndrome; myofacial pain syndrome (MPS); Shigellosis; asthma; adult respiratory distress syndrome; inflammatory bowel disease; Crohn's disease; psoriasis; eczema; ulcerative colitis; glomerular nephritis; scleroderma; chronic thyroiditis; Grave's disease; Ormond's disease; autoimmune gastritis; myasthenia gravis; autoimmune hemolytic anemia; autoimmune neutropenia; thrombocytopenia; pancreatic fibrosis; chronic active hepatitis including hepatic fibrosis; acute and chronic renal disease; renal fibrosis, irritable bowel syndrome; pyresis; restenosis; cerebral malaria; stroke and ischemic injury; neural trauma; Alzheimer's disease; Huntington's disease; Parkinson's disease; acute and chronic pain; allergies, including allergic rhinitis and allergic conjunctivitis; cardiac hypertrophy, chronic heart failure; acute coronary syndrome; cachexia; malaria; leprosy; leishmaniasis; Lyme disease; Reiter's syndrome; acute synovitis; muscle degeneration, bursitis; tendonitis; tenosynovitis; herniated, ruptured, or prolapsed intervertebral disk syndrome; osteopetrosis; thrombosis; silicosis; pulmonary sarcosis; bone resorption diseases, such as osteoporosis or multiple myeloma-related bone disorders; cancer, including but not limited to metastatic breast carcinoma, colorectal carcinoma, malignant melanoma, gastric cancer, and non-small cell lung cancer; graft-versus-host reaction; and auto-immune diseases, such as multiple sclerosis, lupus and fibromyalgia; AIDS and other viral diseases such as Herpes Zoster, Herpes Simplex I or II, influenza virus, Severe Acute Respiratory Syndrome (SARS) and cytomegalovirus; and diabetes mellitus. In addition, the methods of the embodiments can be used to treat proliferative disorders (including both benign and malignant hyperplasias), including acute myelogenous leukemia, chronic myelogenous leukemia, Kaposi's sarcoma, metastatic melanoma, multiple myeloma, breast cancer, including metastatic breast carcinoma; colorectal, carcinoma; malignant melanoma; gastric cancer; non-small cell lung cancer (NSCLC); bone metastases, and the like; pain disorders including neuromuscular pain, headache, cancer pain, dental pain, and arthritis pain; angiogenic disorders including solid tumor angiogenesis, ocular neovascularization, and infantile hemangioma; conditions associated with the cyclooxygenase and lipoxygenase signaling pathways, including conditions associated with prostaglandin endoperoxide synthase-2 (including edema, fever, analgesia, and pain); organ hypoxia; thrombin-induced platelet aggregation; and protozoal diseases.

In some embodiments, the subject is a human.

The terms "therapeutically effective amount," as used herein, refer to an amount of a compound sufficient to cure, ameliorate, slow progression of, prevent, or reduce the likelihood of onset of the identified disease or condition, or to exhibit a detectable therapeutic, prophylactic, or inhibitory effect. The effect can be detected by, for example, the assays disclosed in the following examples. The precise effective amount for a subject will depend upon the subject's body weight, size, and health; the nature and extent of the condition; and the therapeutic or combination of therapeutics selected for administration. Therapeutically and prophylactically effective amounts for a given situation can be determined by routine experimentation that is within the skill and judgment of the clinician.

Also disclosed is the use of a therapeutically effective amount of a Compound I salt (e.g. tosylate) or a pharmaceutical composition thereof as described herein in the preparation of a medicament for treating a fibrotic condition. In some such embodiments, the use further comprises identifying the subject as having or at risk of having said fibrotic condition. In some such embodiments, the fibrotic condition is selected from the group consisting of pulmonary fibrosis, dermal fibrosis, pancreatic fibrosis, liver fibrosis, and renal fibrosis. In some embodiments, the fibrotic condition is idiopathic pulmonary fibrosis. In some embodiments, the subject receiving such treatment is a human being.

In another aspect, a package or kit is provided including a Compound I salt (e.g. tosylate) or a pharmaceutical composition thereof as described herein, optionally in a container, and a package insert, package label, instructions or other labeling according to a method or use described herein.

It will be appreciated that the invention provides a Compound I salt (e.g. tosylate) or a pharmaceutical composition thereof as described herein for use in treating a patient with a disease or condition described herein (e.g. idiopathic pulmonary fibrosis) or a patient who would benefit from administration of such a compound according to any of the treatment regimes as described above with respect to the methods of the invention for administering a Compound I salt (e.g. tosylate) or a pharmaceutical composition thereof as described herein. A Compound I salt (e.g. tosylate) or a pharmaceutical composition thereof as described herein is packaged and presented for use in a treating a patient with a condition or disease described herein (e.g., idiopathic pulmonary fibrosis) or a patient who would benefit from administration of such a compound according to such treatment regimes. A Compound I salt (e.g. tosylate) or a pharmaceutical composition thereof as described herein is administered to the patient in accordance with the treatment regimes as described above.

It will be appreciated that the invention provides the use of a Compound I salt (e.g. tosylate) or a pharmaceutical composition thereof as described herein in the manufacture of a medicament for treating a patient with a disease or condition described herein (e.g., idiopathic pulmonary fibrosis) or a patient who would benefit from administration of such a compound according to any of the treatment regimes as described above with respect to any of the methods. The medicaments manufactured according to this aspect of the invention are for use in treating a patient with a disease or condition described herein (e.g., idiopathic pulmonary fibrosis) or a patient who would benefit from administration of such a compound in accordance with such treatment regimes. The medicament so manufactured is administered to the patient in accordance with the treatment regimes as described above.

In respect of the aspects of the invention relating to a Compound I salt (e.g. tosylate) or a pharmaceutical composition thereof as described herein for use in treating a patient, and to use of a Compound I salt (e.g. tosylate) or a pharmaceutical composition thereof as described herein in the manufacture of a medicament for treating a patient, the preferences expressed with respect to the preferred embodiments of the aspects of the invention relating to methods for administering such compounds to treat a patient with apply in the same way.

### EXAMPLES

The following examples are provided for illustration and are not intended to limit the scope of the invention.

### Example 1 - Salt Screening

The pKa value of Compound I free base was calculated (ACD Labs pKa DB, v10.0) as 2.12 ± 0.33. Therefore, Compound I free base was considered as a very weak base that could be protonated by strong acids. Experimental determination of the pKa resulted in a value of 2.5. A sample of Compound I free base was analyzed by powder x-ray diffraction and found to be crystalline without any readily detectable amorphous content. Mass loss due to decomposition was observed at temperatures above 220 °C, with no other features in a thermogravimetric investigation other than a small mass loss of about 0.1% below 220 °C, indicating that the crystalline form of Compound I free base was neither a solvate nor a hydrate. Investigation of the hygroscopic properties of the crystalline form of Compound I free base showed that the maximum water up-take at 95% relative humidity was less than 0.1%. Therefore, it was concluded that the crystalline form of Compound I free base was not hygroscopic and the amount of adsorbed water was very small. DSC analysis showed a single melting peak at 293.4 °C (onset temperature).

A screening program was performed for salts of Compound I, including salt and co-crystal formers adipic acid, benzenesulfonic acid, citric acid, ethanedisulfonic acid, fumaric acid, glutaric acid, glycolamide, hydrobromic acid, hydrochloric acid, L-lactamide, L-malic acid, maleic acid, malonic acid, methanesulfonic acid, 1,5-naphthalenedisulfonic acid, nicotinic acid, nitric acid, oxalic acid, phosphoric acid, saccharin, sorbic acid, succinic acid, sulfuric acid, L-tartaric acid, urea, and p-toluenesulfonic acid.

As an initial experiment, eighteen formers (including two charge ratios of HCl) and four solvent systems were used under 76 conditions in a first-tier screening. These results are summarized in Table 1. As gels were generated in most of the screening experiments, a second-tier screening was conducted at 5 °C using five strong acids (including two charge ratios of HCl) and two different solvent systems for a total of 12 conditions. These results are summarized in Table 2. From all the screening experiments, the crystalline hits were isolated and characterized by X-ray powder diffraction (XRPD), thermo-gravimetric analysis (TGA), and differential scanning calorimetry (DSC). The salt stoichiometry was determined using proton nuclear magnetic resonance (¹H NMR) or HPLC combined with ion chromatography (IC). Meanwhile, a hemi-THF solvate of the free base was also identified.

**Table 1**

| **Acid** | **Solvent** | | | |
|---|---|---|---|---|
| | **Acetone** | **ACN/H₂O (9:1, v/v)** | **DCM** | **MeOH** |
| Hydrochloric acid (1:1) | Gel | - | Gel | - |
| Hydrochloric acid (2:1) | Gel | Gel | Gel | Gel |
| Sulfuric acid | Gel | Gel | Gel | - |
| Phosphoric acid | - | - | - | - |
| Methanesulfonic acid | Gel | - | Gel | - |
| Benzenesulfonic acid | Gel | - | Gel | - |
| Toluenesulfonic acid | Mono-Tosylate Type A | Gel | Mono-Tosylate Type A | - |
| Adipic acid | - | - | - | - |
| Fumaric acid | - | - | - | - |
| Succinic acid | - | - | - | - |
| Maleic acid | - | - | Gel | - |
| L-malic acid | - | - | Gel | - |
| Malonic acid | - | - | Gel | - |
| Sorbic acid | - | - | - | - |
| Glutaric acid | - | - | Gel | - |
| Nicotinamide | - | - | - | - |
| L-lactamide | - | Gel | - | - |
| Urea | - | - | - | - |
| Glycolamide | - | - | - | - |

**Table 2**

| **Acid** | **Solvent** | |
|---|---|---|
| | **THF** | **EtOAc** |
| Hydrochloric acid (1:1) | - | Gel |
| Hydrochloric acid (2:1) | Gel | Gel |
| Sulfuric acid | Sulfate Type A and Free Base Type A | Gel |
| Phosphoric acid | - | Phosphate Type A and Free Base Type A |
| Methanesulfonic acid | Mesylate Type A | - |
| Benzenesulfonic acid | Besylate Type A | - |

About 15 mg of free base was dispersed in the selected solvent in a glass vial, and the corresponding acid was added with a molar charge ratio of 1:1 (for HCl, two ratios of 1:1 and 2:1 were used, considering the two basic functional groups existing in the free base). The mixtures of free base and acid were stirred at room temperature for 4 days. Clear solutions were transferred to slurry at 5 °C for 3 days and the final clear solutions after cooling were allowed to evaporate slowly at room temperature. The resulting solids in each step were isolated and analyzed by XRPD.

A considerable number of experiments from the first-tier screening resulted in gel formation. Therefore, second-tier screening experiments were set up at 5 °C using five strong acids and two different solvents. A total of five crystalline hits were observed.

A stock solution of Compound I free base in methanol-dichloromethane 9:1 was prepared. Aliquots of the stock solution were pipetted into each well of a microtiter plate, and subsequently aliquots of acid stock solution were added to each well. The mixtures were subjected to each of eight different experimental conditions: evaporation of solvents from the initial solvent mixture under nitrogen, suspension equilibration in acetonitrile, suspension equilibration in ethanol, suspension equilibration in ethyl acetate, suspension equilibration in ethyl acetate-dichloromethane, suspension equilibration in heptane-THF 1:1, suspension equilibration in isopropanol, suspension equilibration in methanol-water 4:1, and suspension equilibration in THF-formic acid 9:1. Screening was carried out with 12 different acids under eight different conditions; *i.e.* 96 experiments were performed. From these initial solvent mixtures, the solvents were evaporated under a slight nitrogen flow at room temperature. For suspension equilibration conditions, the suspension equilibration was performed at 25 °C for two days before the added solvents were evaporated, again under slight nitrogen flow. Within about 18 hours, solid residues were obtained and then examined by Raman microscopy.

The benzenesulfonate (besylate), ethanedisulfonic (edisylate), 1,5-naphthalenedisulfonic (napadisylate), and p-tolunesulfonic (tosylate) salts of Compound I were also prepared. Using a similar strategy as described above, these salts were prepared by evaporation from a solution of free base and acid in a solvent (*e.g*., methanol-dichloromethane 1:1, ethyl acetate-dichloromethane 1:2, isopropanol, and the like). If necessary, an amorphous salt produced in this way was crystallized by stirring the amorphous material in an appropriate solvent (*e.g*., ethyl acetate, acetonitrile, and the like). The solid residues were characterized by ¹H-NMR spectroscopy, X-ray diffraction, TG-FTIR, DVS, DSC, and aqueous solubility at 25 °C. ¹H-NMR analysis indicated 2:1 free base:acid stoichiometry for the napadisylate and edisylate salts; accordingly, these salts are more properly referred to as hemi-napadisylate and hemi-edisylate, respectively.

In many of the experiments, the same crystalline form of Compound I free base was found (Form A, Figure 1). This form was fairly stable and showed a stronger tendency to crystallize than any of the investigated salt or co-crystal systems. Many of the obtained samples were predominantly amorphous, although crystal forms were obtained with systems that included the strong acids, namely hydrobromic acid, hydrochloric acid, nitric acid and sulfuric acids.

Table 3 below shows solubility data for Compound I salt forms in pure water at 25°C after one hour (not all samples), and after 24 hours of equilibration time. PXRD measurements of the solid residues after solubility testing showed that conversion into the free base typically occurred within one hour.

**Table 3**

| **Salt form** | **S [mg/ml] 1 hr** | **pH** | **S [mg/ml] 24 hrs** | **pH** |
|---|---|---|---|---|
| mono-HBr amorphous | n.a. | n.a. | 0.62 | 1.6 |
| mono-HBr crystalline | n.a. | n.a. | 0.31 | 1.9 |
| di-HBr crystalline | 0.54 | 1.6 | 0.48 | 1.6 |
| mono-HCl amorphous | n.a. | n.a. | 0.70 | 1.6 |
| mono-HCl crystalline | n.a. | n.a. | 0.42 | 1.6 |
| di-HCl crystalline | 1.14 | 1.3 | 0.76 | 1.5 |
| mono-nitrate amorphous | n.a. | n.a. | 0.33 | 1.9 |
| mono-nitrate crystalline | 0.42 | 1.8 | 0.38 | 1.8 |
| mono-sulfate amorphous | 1.26 | 1.2 | 0.84 | 1.4 |
| mono-sulfate crystalline | 1.73 | 1.1 | 1.54 | 1.2 |
| besylate | 1.26 | 1.50 | 1.20 | 1.50 |
| hemi-edisylate | 1.09 | 1.42 | 0.99 | 1.48 |
| hemi-napadisylate | 2.76 | 1.53 | 1.83 | 1.53 |
| mono-tosylate | 2.54 | 1.47 | 1.24 | 1.53 |

In Table 4 below some of the physicochemical properties of the free base and amorphous salt forms are presented.

**Table 4**

| **Solid form** | **Solubility (24 hours)** | **H₂O uptake at 95% relative humidity** | **T_{g}** | **TG-FTIR** |
|---|---|---|---|---|
| free base | 0.057 mg/ml | < 0.1% | 68°C | < 0.1% (200°C) |
| sulfate 1:1 | 0.84 mg/ml | 32.6% | 96°C | ∼ 1% (200°C) |
| hydrochloride 1:1 | 0.70 mg/ml | 40.0% | 103°C | ∼ 1% (160°C) |
| hydrobromide 1:1 | 0.62 mg/ml | 28.8% | 108°C | ∼ 2% (180°C) |
| nitrate 1:1 | 0.33 mg/ml | 24.6% | 95°C, dec | ∼ 1% (100°C) |

In Table 5 below some of the physicochemical properties of the crystalline salt forms are presented.

**Table 5**

| **Solid form** | **Solubility (24 hours)** | **H₂O uptake at 95% relative humidity** | **Melting point** | **TG-FTIR** |
|---|---|---|---|---|
| free base | 0.057 mg/ml | < 0.1% | ∼ 276°C | < 0.1% (200°C) |
| sulfate | 1.54 mg/ml | 34% | 171°C | < 0.3% (200°C) |
| di-hydrochloride | 0.76 mg/ml | 54% | ~161°C, dec. | ∼ 7% EtOAc |
| di-hydrobromide | 0.48 mg/ml | 47% | ∼ 148°C | ∼ 8% EtOAc |
| nitrate 1:1 | 0.38 mg/ml | 27% | ~119°C, dec. | ∼ 1.5% (150°C) |
| besylate | 1.20 mg/ml | ~24% | 186 °C | -0.24% (250 °C) |
| edisylate | 0.99 mg/ml | ~32% | ~210 °C | -1.8% (250 °C) |
| napadisylate | 1.83 mg/ml | -18% | ∼234 °C | ~3.3% (250 °C) |
| mono-tosylate | 1.24 mg/ml | 2.8% | ~207 °C | <0.2% (250 °C) |

The sulfate salt exhibited good thermal stability, and was further characterized by a clear melting point in the region of 170°C to 180°C. It was produced in a crystalline form that was easy to dry, as the residual solvent content of the obtained sample was low. The particle size distribution and crystal habits found were acceptable.

All produced salts showed enhanced aqueous solubility; however, none of the solid salt forms investigated in this Example was stable in the aqueous phase. PXRD measurements of the solid residues after solubility testing showed that conversion into the free base typically occurred within one hour.

All of the produced salts, whether amorphous or crystalline, were at least partially deliquescent at relative humidities above about 75%. These salts should be stored at low relative humidity, preferably below 50%, and in air tight containers to prevent water adsorption. In addition, wet granulation with water containing solvents should be avoided.

### Example 2 - Compound I Hydrochloride Salt

To a solution of Compound I free base in dioxane was added aqueous HCl (1.1 equivalents). After stirring for 0.5 hours the solvent was removed under reduced pressure and the residue was dried in vacuum to afford the hydrochloride salt of Compound I.

The identity of the hydrochloride salt was confirmed by H-NMR spectroscopy, Raman spectroscopy, and elemental composition analysis. H-NMR and TG-FTIR analysis revealed that the sample contained a significant amount of ethyl acetate, and the theoretically expected content was adjusted to account for this observation. It was suspected that an ethyl acetate solvate was obtained.

Water sorption measurements of the hydrochloride salts showed that both investigated salts strongly adsorb water at high relative humidity. The maximum water uptake for the amorphous salt was about 40% whereas the maximum water uptake for the crystalline di-hydrochloride salt was higher, with about 54% uptake.

### Example 3 - Compound I Hydrobromide Salt

The amorphous mono-hydrobromide salt was produced by dissolving Compound I free base in a mixture of methanol-dichloromethane 2:1 and adding one equivalent of HBr in the form of a 2 M aqueous HBr solution. The solvents were removed by rotary evaporation at 40°C and a solid white residue was obtained. Further drying was carried out under vacuum at 40°C.

The crystalline hydrobromide was obtained by suspension equilibration in ethyl acetate. Crystallization was favored if two equivalents of HBr were used. To about 200 mg of free base in 2.0 ml ethyl acetate, two equivalents of HBr were added in the form of a 33% w/w solution of HBr in acetic acid. After stirring at room temperature the salt began to crystallize, and an easy-to-handle white powder was obtained.

The identity of the hydrobromide salt was confirmed by H-NMR spectroscopy, Raman spectroscopy, and elemental composition analysis.

Powder X-ray diffraction patterns of the hydrobromide salt samples were found to show considerable variation.

Water sorption measurements of the hydrobromides show that both investigated salts strongly adsorbed water at high relative humidity. The maximum water uptake for the amorphous salt was about 29% whereas the maximum water uptake for the crystalline di-hydrobromide salt was about 47%. Visual inspection of both samples after the DVS test revealed a slightly brownish discoloration that had deliquesced during the measurement.

### Example 4 - Compound I Sulfate Salt

The sulfate salt was produced as a monosalt, and therefore, the term hydrogensulfate is more accurate; however, for simplicity salt is generally referred to herein as the sulfate. The amorphous sulfate salt was produced by dissolving Compound I free base in a mixture of methanol-dichloromethane 2:1 and adding one equivalent of sulfuric acid (95-97% concentrated). From the clear solution the solvents were removed by rotary evaporation at 40°C and a solid white residue was obtained. Further drying was carried out under vacuum at 40°C for about 18 hours. The crystalline sulfate salt was produced by suspension equilibration of the amorphous salt form in ethyl acetate.

The identity of the sulfate salt was confirmed by H-NMR spectroscopy, Raman spectroscopy, and elemental composition analysis.

Water sorption measurements of the sulfate salts show that both investigated salts strongly adsorbed water at high relative humidity. The maximum water uptake for the amorphous salt was about 33% whereas the maximum water uptake for the crystalline salt was about 35%.

### Example 5 - Compound I Nitrate Salt

The amorphous nitrate salt was produced by dissolving Compound I free base in a mixture of methanol-dichloromethane (about 2:1) and adding one equivalent of nitric acid in the form of a 70% aqueous solution. From the clear solution the solvents were removed by rotary evaporation at 40°C and a solid white residue was obtained. Further drying was carried out under vacuum at 40°C for about 18 hours. The nitrate spontaneously crystallized when amorphous nitrate was suspended in ethyl acetate. The dried solid was essentially free of residual ethyl acetate.

The identity of the crystalline nitrate salt was confirmed by H-NMR spectroscopy, Raman spectroscopy, and elemental composition analysis.

Water sorption measurements of the nitrate salts showed that both investigated salts strongly adsorbed water at high relative humidity. The maximum water uptake for the amorphous salt was about 25% whereas the maximum water up-take for the crystalline nitrate was about 27%. In contrast to the hydrochloride, hydrobromide, and sulfate salts, the crystalline nitrate did not show an initial water uptake at the beginning of the measurement program, which was set to 50% relative humidity.

### Example 6 - Compound I Besylate Salt

The amorphous besylate salt was produced by dissolving Compound I free base in a mixture of methanol - dichloromethane (about 1:1) and adding about two equivalents of benzenesulfonic acid. From the clear solution thus obtained, the solvents were removed by rotary evaporation at 42°C and a solid white residue was obtained. To this residue was added ethyl acetate, and the resulting white suspension was stirred at room temperature for about 20 hours. Alternately, acetonitrile was added to the amorphous besylate salt residue, and the resulting white suspension was stirred at room temperature for about 20 hours.

The identity of the crystalline 1:1 besylate salt was confirmed by H-NMR spectroscopy. Further characterization comprised powder X-ray diffraction, TG-FTIR, DVS, DSC, and aqueous solubility at 25°C.

The hygroscopic nature of the besylate salt was examined by dynamic vapor sorption analysis (DVS). The adsorbed water at 95% relative humidity was about 24%; however, the sample did not deliquesce completely. Below 80% relative humidity not much water was adsorbed. About 6% water was still adsorbed at the end of the cycle as the water adsorption was not reversible within the timeframe of observation.

### Example 7 - Compound I Hemi-Edisylate Salt

The crystalline hemi-edisylate salt was produced by mixing two stock solutions as follows: a stock solution of ethanedisulfonic acid in isopropanol was added to solid free base, and the mixture was stirred at room temperature for three days before the solid was separated by filtration. The obtained solid was dried at 40°C under vacuum for about three hours.

H-NMR spectroscopy confirmed the 2:1 salt of Compound I with ethanedisulfonic acid. Further characterization comprised powder X-ray diffraction, TG-FTIR, DVS, DSC, and aqueous solubility at 25°C.

The hygroscopic nature of the hemi-edisylate salt was examined by dynamic vapor sorption analysis (DVS). The amount of adsorbed water at 95% relative humidity was about 32%. The water adsorption was not reversible within the timeframe of observation.

### Example 8 - Compound I Hemi-Napadisylate Salt

All attempts to obtain a crystalline napadisylate salt with a 1:1 stoichiometry failed, so further crystallization experiments were directed towards preparation of a crystalline hemi-napadisylate salt. The crystalline hemi-napadisylate salt could be produced successfully by crystallization from a mixture of DCM and ethyl acetate. Generally, the free base was dissolved in DCM and the naphthalenedisulfonic acid was dissolved in ethyl acetate, the solutions were mixed and DCM was allowed to evaporate either at room temperature or at a slightly elevated temperature, e.g., at 60°C.

The crystalline hemi-napadisylate salt was produced by mixing stock solutions as follows: naphthalene-disulfonic acid in ethyl acetate was mixed with solid free base (2 equivalents) dissolved in DCM. The mixture was heated to 60°C and the DCM was distilled off while stirring at 60°C for about two hours before the heater was turned off and the system was allowed to cool to room temperature. Stirring was continued overnight at room temperature while keeping the vial open, and on the next day the suspension was filtered. The obtained solid was dried under vacuum at 40°C for about three hours. H-NMR spectroscopy confirmed the 2:1 salt of Compound I with naphthalene-1,5-disulfonic acid; however, the H-NMR reveals the presence of DCM and ethyl acetate as residual solvents. Further characterization comprised powder X-ray diffraction, TG-FTIR, DVS, DSC, and aqueous solubility at 25°C.

The hygroscopic nature of the hemi-napadisylate salt was examined by dynamic vapor sorption analysis (DVS). The adsorbed water at 95% relative humidity was about 17.8%. The water adsorption was not reversible within the timeframe of observation.

### Example 9 - Compound I Tosylate Salt

Both the mono-tosylate salt and the di-tosylate salt crystallized well and were produced by several suitable processes. For example, amorphous tosylate salt was produced by evaporation of a solution of free base and toluenesulfonic acid in methanol - dichloromethane 1:1, then ethyl acetate was added and the resulting suspension was stirred until crystallization was complete.

The solid products were generally easy to isolate and to dry. In one experiment, the mono-tosylate salt was produced and a basic characterization was performed. H-NMR spectroscopy confirmed the ratio of free base to toluenesulfonic acid as 1:1. Additionally, the mono-tosylate salt was characterized by powder X-ray diffraction, TG-FTIR, DVS, DSC, and aqueous solubility at 25°C. The X-ray diffraction pattern of the mono-tosylate salt is shown in Figure 8 with peaks at diffraction angle 2θ values at 10.9214°, 13.2780°, 15.3605°, 16.9425°, 17.7356°, 18.2003°, 20.5139°, 23.2091°, 23.8569°, 24.7278°, 25.6871°, 26.6843°, 27.6274°, 29.1166°, and 30.5294°.

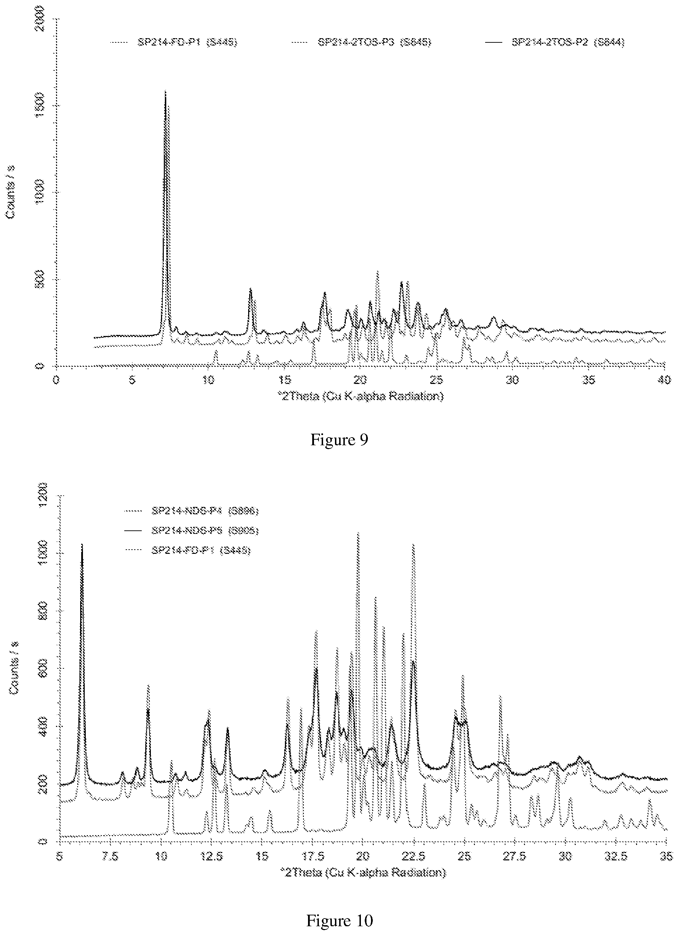

A crystalline di-tosylate salt was also produced by suspension experiments with the amorphous di-tosylate in ethyl acetate or acetonitrile. The PXRD patterns of the two produced samples are depicted in Figure 9. H-NMR spectroscopy confirmed the ratio of free base to toluenesulfonic acid as 1:2. However, H-NMR revealed the formation of solvates in both experiments. The sample obtained from the suspension experiment in ethyl acetate appeared to be an ethyl acetate hemisolvate, and an acetonitrile hemisolvate was probably obtained from the suspension experiment with acetonitrile.

The hygroscopic nature of the mono-tosylate salt was examined by dynamic vapor sorption analysis (DVS). The adsorbed water at 95% relative humidity was about 2.8%. The water adsorption was essentially reversible within the timeframe of observation and the adsorbed water was removed at the end of the cycle.

### Example 10 - Preparation of Compound I Mono-Tosylate Salt with Narrow Particle Size Distribution

Compound I mono-tosylate anhydrate (Type A) was successfully prepared via anti-solvent crystallization at room temperature in methanol (MeOH) and methyl tert-butyl ether (MTBE, anti-solvent) with desired quality attributes.

Two batches were first synthesized in MeOH/MTBE via magnetic stirring, with addition times of 2 and 6 hours, respectively. Both afforded mono-tosylate Type A with the desired quality attributes including particle size distribution, with a volumetric efficiency of ~16 L/kg and yield of > 85%. A demonstration batch was also prepared via overhead stirring with addition over 6 hours. The batch was characterized via X-ray powder diffraction (XRPD), thermo-gravimetric analysis (TGA), differential scanning calorimetry (DSC), polarized light microscopy (PLM), particle size analyzer, proton nuclear magnetic resonance (¹H NMR), gas chromatography (GC), and HPLC. The particles were cubic and about 50 µm in size before filtration. However, agglomeration was observed after washing and drying, which led to a measured D90 of 150.0 µm.

To investigate the possible effect of seed size and addition time on the particle size of the final product, different batches of seeds were used to prepare another two batches via overhead stirring with addition time over 2 hours. It was confirmed via PLM that agglomeration occurred for both batches after washing and drying. Seeding with larger particles resulted in a bimodal particle size distribution, which was likely caused by the co-occurrence of crystal growth and nucleation during the process. Thus, small or milled seeds were preferred for the process.

The approximate solubility of mono-tosylate Type A was measured in MeOH/MTBE at room temperature. The results showed that solubility ranges of mono-tosylate Type A were 264.0-348.0 mg/mL in MeOH, 9.3-18.6 mg/mL in MeOH/MTBE (v/v, 1:2) and < 5.4 mg/mL in MeOH/MTBE (v/v, 1:3). To achieve appropriate volumetric efficiency and yield, the starting concentration in MeOH was 250 mg/mL and the final ratio of the anti-solvent crystallization process in MeOH/MTBE (v/v) was 1:3.

Two batches of mono-tosylate Type A were produced via anti-solvent crystallization in MeOH/MTBE (charge molar ratio of 1:1), with different addition rates of anti-solvent. The products were characterized by XRPD, DSC, GC, PLM, and PSD. The PSD data showed a unimodal distribution with desired D90 (~ 30 µm to less than 100 µm), possibly due to the milling effect of magnetic stirring.

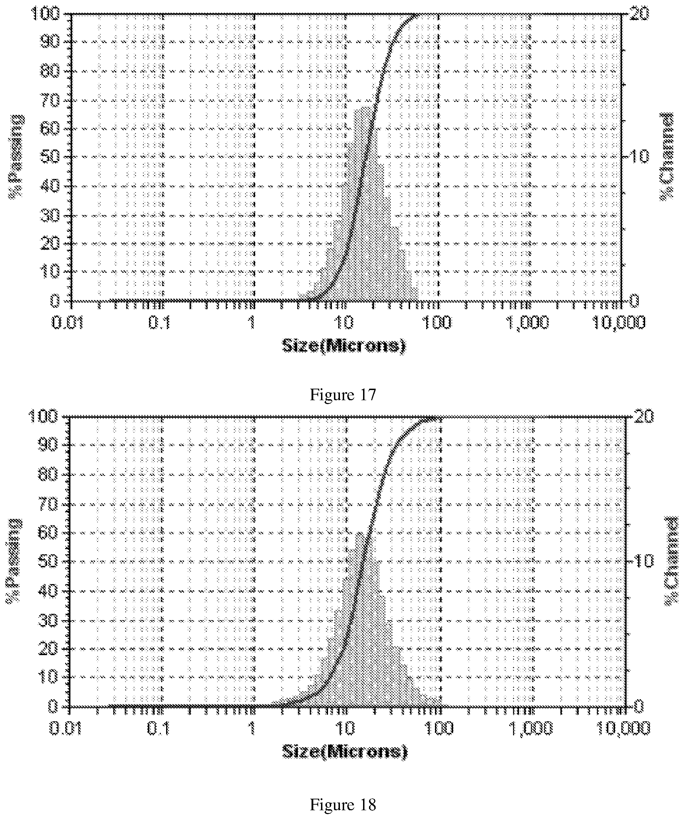

The residual solvent level and crystallinity were found to be similar regardless of anti-solvent addition time (2 or 6 hours). Particle morphology and particle size distribution produced via overhead stirring were also found to be similar regardless of anti-solvent addition time (2 or 6 hours). The details are provided in Table 6 and Figures 17 and 18.

Process parameters and characterization data are summarized in Table 6. The two batches with D90 of ~ 30 µm were ascribed to the milling effect of magnetic stirring. It was also found that addition time of 2 or 6 hours did not have a large impact on the dispersion.

**Table 6**

| Batch | 1 | 2 | 3 | 4 | 5 |
|---|---|---|---|---|---|
| Agitation method | Magnetic stir | Magnetic stir | Overhead stir | Overhead stir | Overhead stir |
| Addition time (hours) | 6 | 2 | 6 | 2 | 2 |
| Mv of seed (µm) | 91.09 | 91.09 | 18.65 | 18.65 | 105.1 |
| Melting point (onset, °C) | 204.4 | 204.9 | 205.2 | 204.6 | 204.3 |
| Mv (µm) | 91.09 | 91.09 | 18.65 | 18.65 | 105.1 |
| D90 (µm) | 32.56 | 33.76 | 150.0 | 186.6 | 314.6 |
| Morphology | Irregular particles | Irregular particles | Bulk particles | Bulk particles | Cubic particles |

In summary, an anti-solvent crystallization process in MeOH/MTBE at room temperature produced mono-tosylate Type A with desired quality attributes (purity ≥ 99.8 area%, yield of ~ 90%, residual MeOH/MTBE level lower than the ICH limit: MeOH 3000 ppm, MTBE 5000 ppm). The crystallization process with parameters to prepare Compound I mono-tosylate Type A is summarized below:
1) An initial solution of Compound I and p-toluenesulfonic acid in MeOH was prepared with a concentration of 250 mg/mL at room temperature, with a charge molar ratio of 1:1.
2) The solution was supersaturated by adding MTBE, to produce a volumetric ratio of 3:1 MeOH/MTBE.
3) To this solution was added 5% seeds of mono-tosylate Type A, and the mixture was aged for 10~30 minutes.
4) To this solution was added MTBE over 2-6 hours to produce a final volumetric ratio of 1:3 (MeOH/MTBE), and the solution was then aged 1-5 hours before filtration.
5) The filtered product was washed with MTBE, and dried in a vacuum oven at 50 °C overnight.

### Example 11 - Preparation of Compound I Mono-Tosylate Salt at 15 Gram Scale

Compound I mono-tosylate anhydrate (Type A) was prepared as described in Example 10, scaled up to 15 grams with a yield of 91.0%. The scaled-up product was characterized by XRPD, TGA, DSC, GC, PLM, and PSD. Bulk crystals were obtained with a D90 of 150.0 µm.

The particle size increased when compared with previous 1.5-g batches. Agglomeration was observed after washing and drying as determined via PLM imaging. It is also possible that the solvent system (MeOH/MTBE) was suitable for particle growth.

### Example 12 - Effect of Seed Size on Compound I Mono-Tosylate Salt Particle Size

Two batches of mono-tosylate Type A were produced over 2 hours of anti-solvent addition time, with different seed sizes (mean diameter Mv of 18.65 µm and 105.1 µm). The final dry products were characterized by XRPD, DSC, GC, PLM, and PSD.

The smaller seeds gave crystals with an average particle size ~ 50 µm, but agglomeration led to an increase of the D90 during the solvent wash with MTBE and drying process. When larger particles were used as seeds, a bimodal particle size distribution was observed, possibly related to the co-occurrence of crystal growth and nucleation during the process.

### Example 13 - Synthesis of Compound I Tosylate Salt

A process for the formation of mono- and di-tosylate salts of Compound I was developed and a batch was performed to successfully produce the mono-tosylate salt.

### Step 1: Synthesis of 2-chloro-N-methyl-3-nitropyridin-4-amine

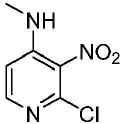

A reactor was charged with 2,4-dichloro-3-nitropyridine and 3.0 volumes of DMF. The solution was stirred at 20-25 °C until a clear solution was obtained. The solution was then cooled to 0-5 °C, and 2.1 equivalents of 40% methylamine in water were slowly added over at least 2 hours at 0-5 °C. The reaction mixture was stirred for at least 2 hours at 0-5 °C until conversion to the product was 95% (as measured by HPLC). The reaction mixture was diluted by slowly adding 10 volumes of water over at least 30 minutes at 0-5 °C. The obtained suspension was stirred for at least 60 minutes at 0-5 °C. The precipitate was collected by filtration, and the filter cake was rinsed via the reactor with 10 volumes of water at 0-5 °C. The damp filter cake was then dried in a flow of dry nitrogen to yield 2-chloro-N-methyl-3-nitropyridin-4-amine in 78% yield.

### Step 2: Synthesis of 2-chloro-N4-methylpyridine-3,4-diamine

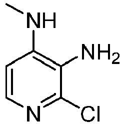

A reactor was charged with catalyst [2% Pt on charcoal, 59 %wt. water] (0.0004 equivalents Pt), damp 2-chloro-N-methyl-3-nitropyridin-4-amine from step 1 and 9.4 volumes of THF. The solution was stirred, and then the suspension was transferred from the glass-reactor to an autoclave. The line was rinsed with 1.2 volumes of THF into the autoclave, and the autoclave was purged with nitrogen for 15 minutes at 50 rpm, followed by hydrogen for 15 minutes at 150 rpm. The autoclave was closed, and the hydrogen pressure was adjusted to 2 bar at 20-30 °C. The reaction mixture was stirred for 4-8 hours at 2 bar and 20-30 °C.

Next, the autoclave was released to atmospheric pressure and purged with nitrogen for at least 15 minutes. Conversion to the product was verified by HPLC, and then the catalyst was removed by filtration. The filtered catalyst was rinsed with 1.3 volumes of THF and the filtrates were combined. The combined filtrates were charged to a second reactor via a particle filter, and the line was rinsed with 0.5 volumes of THF. The solution was concentrated to a final volume of 2.5 volumes by distillation under reduced pressure at 40-45 °C.

The solution was then diluted with 10 volumes of THF in portions while concentrating the solution to a final volume of 2.5 volumes by distillation under reduced pressure at 45-50 °C. The reactor was purged with nitrogen to atmospheric pressure, and 5.0 volumes of heptane were added to the residue at 40-50 °C. The reaction mixture was cooled over 2 hours to 20-25 °C, and stirring was continued for 1 hour. The reaction mixture was then further cooled to 0-5 °C over 1 hour, and stirring was continued for 1 hour. The precipitated product was collected by filtration, rinsed via the reactor with 5.0 volumes of heptane, and the damp filter cake was dried in a vacuum drying oven at max. 40 °C until loss on drying was ≤ 2 % weight, giving 2-chloro-N⁴-methylpyridine-3,4-diamine in 85% yield.

### Step 3: Synthesis of 1-methyl-1,5-dihydro-4H-imidazo[4,5-c]pyridin-4-one

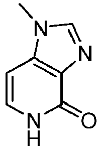

A reactor was charged with 2-chloro-N⁴-methylpyridine-3,4-diamine and 4 volumes of formic acid. The reaction mixture was heated to smooth reflux within one hour, and reflux was maintained for 6 hours. The reaction mixture was then cooled to approximately 60 °C, and conversion to the product was verified by HPLC.

The reaction mixture was then concentrated by distillation under reduced pressure at 60-80 °C to a final volume of 2 volumes. The temperature of the solution was adjusted to 60 °C, maintaining the temperature above 50 °C to avoid precipitation.

Next, a second reactor was charged with 10 volumes of acetone, and heated to gentle reflux. The product solution from the first reactor was slowly transferred to the acetone in the second reactor over 20 minutes, and the line was rinsed with approximately 0.05 volumes of formic acid. Reflux of the obtained suspension was maintained for 15 minutes. The slurry was cooled to 0 °C within 1 hour, and stirring was continued for 1 hour at that temperature. The precipitate was collected by filtration, and the filter cake was rinsed via the reactor with 3.7 volumes of cold acetone at 0-10 °C. The filter cake was dried in a flow of dry nitrogen or in a vacuum drying oven at 50 °C until loss on drying was ≤ 2% of weight, giving 1-methyl-1,5-dihydro-4*H*-imidazo[4,5-c]pyridin-4-one in 95% yield.

### Step 4: Synthesis of 1-methyl-5-(4-(trifluoromethoxy)phenyl)-1,5-dihydro-4H-imidazo[4,5-c]pyridin-4-one

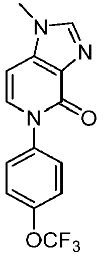

A first reactor (Reactor A) was charged with 1-methyl-1,5-dihydro-4*H-*imidazo[4,5-c]pyridin-4-one (1.0 mol equivalent), Cu(OAc)₂·H₂O (0.1 mol equivalents), and K₂CO₃ (1.1 mol equivalents). The reactor was closed and the atmosphere replaced with nitrogen.

Next, 1-bromo-4-(trifluoromethoxy)benzene (1.5 mol equivalents) and N-methylpyrrolidinone (5.4 volume equivalents) were added, whereupon a suspension was formed. The suspension was stirred until the temperature had fallen again to approximately 20-25 °C and gas evolution had slowed. The reaction mixture was heated to approximately 130-150 °C at which time a blue/green color was observed, changing to dark brown after some time. The reaction was stirred at 130-150 °C for at least 40 hours. Stirring times of 40 hours up to 72 hours were required to reach an acceptable level of conversion. In general, higher reaction temperatures supported faster conversion.

Next, the reaction mixture was cooled to approximately 20-30 °C, and 25% aqueous NH₃ (0.7 volume equivalents) was added, followed by water (3.5 volume equivalents). The resulting suspension was transferred into a second reactor (Reactor B). Additional water was added (18.1 volume equivalents) to the reaction mixture via Reactor A, followed by n-heptane (3.2 volume equivalents). The resulting suspension was cooled to approximately 0-5 °C, and stirred for approximately 2 hours.

The suspension was filtered, and the filter cake was washed with water (9.7 volume equivalents). The filter cake was then dissolved in dichloromethane (14.1 volume equivalents) and transferred back into reactor B. To this solution was added water (5.7 volume equivalents) via the filter, followed by 25% aq. NH₃ (1.6 volume equivalents). The mixture was stirred for approximately 1 hour at approximately 15-25 °C.

Next, the layers were separated, and dichloromethane was added (3.6 volume equivalents) to the aqueous layer. The biphasic mixture was stirred at approximately 15-25 °C for approximately 20-30 minutes. The layers were separated over a period of at least 1 hour, and to the combined organic layers was added a solution of NH₄Cl (2.5 mol equivalents) in water (7.0 volume equivalents). The biphasic mixture was stirred at approximately 15-25 °C for about 20-30 minutes, then the layers were separated over the course of 1 hour.

The lower organic layer was filtered through a particle filter and diluted with toluene (7.1 volume equivalents) via the filter. The organic layer was concentrated under ambient pressure at approximately 80 °C, until no further liquid was seen to evaporate and a precipitate began to form. Toluene was added (16.6 volume equivalents), then concentrated in vacuo, followed by addition of more toluene (7.1 volume equivalents) and again concentrated in vacuo. The suspension was cooled to approximately 0-5 °C, stirred for approximately 2 hours, and filtered. The filter cake was washed with toluene (2.9 volume equivalents), and dried in vacuo at approximately 50 °C until the loss on drying was 0.5% of the weight to give 1-methyl-5-(4-(trifluoromethoxy)phenyl)-1,5-dihydro-4*H*-imidazo[4,5-c]pyridin-4-one as a beige-colored solid in 83.1% yield.

### Step 5: Synthesis of 7-bromo-1-methyl-5-(4-(trifluoromethoxy)phenyl)-1,5-dihydro-4H-imidazo[4,5-c]pyridin-4-one

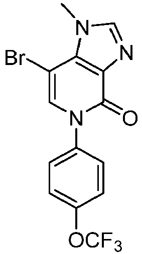

A first reactor (Reactor A) was charged with water (1.8 volume equivalents) and cooled to approximately 0-5 °C, to which was slowly added 96% sulfuric acid (14 mol. equivalents) at approximately 0-20 °C. The temperature of the solution was adjusted to approximately 0-5 °C, and 1-methyl-5-(4-(trifluoromethoxy)phenyl)-1,5-dihydro-4H-imidazo[4,5-c]pyridin-4-one (1.0 mol equivalent) was added in 3-4 portions at approximately 0-5 °C. The temperature of the mixture was adjusted to approximately 0-5 °C, and N-bromosuccinimide (1.0 mol equivalents) was slowly added in 3-4 portions, while maintaining the temperature at approximately 0-5 °C.

The reaction mixture was stirred for about 1 hour at approximately 0-5 °C, and then for an additional 4-16 hours at approximately 0-22 °C. Conversion to the product was confirmed by HPLC, then the reaction mixture was cooled to approximately 0-5 °C.

A second reactor (Reactor B) was charged with water (42.7 volume equivalents) and cooled to approximately 0-5 °C. The reaction mixture from Reactor A was transferred into the pre-cooled water in Reactor B at a temperature below 30 °C over 2 hours. The reaction was rinsed with water (1.6 volume equivalents), and 50% aqueous sodium hydroxide (25 mol. equivalents) was carefully added at approximately 0-30 °C over about 2 hours until the pH reached 2-5.

Next, MTBE (6.5 volume equivalents) was added at approximately 0-20 °C, and the mixture was stirred for about 5 minutes. Additional 50% aqueous sodium hydroxide (2 mol. equivalents) was added at approximately 0-30 °C until the pH of the solution was in the range of 10-14. The reaction was stirred for at least 1.5 hours at approximately 15-25 °C, and then the layers were allowed to separate over a period of at least 1 hour. The suspension was filtered, taking care to capture the product, which accumulated at the interface of the aqueous and organic layers. The filter cake was washed with MTBE (1.7 volume equivalents), water (3.0 volume equivalents), and then MTBE again (3.0 volume equivalents). The product was dried in vacuo at below 50 °C until the loss on drying was ≤ 1% of the weight, giving 7-bromo-1-methyl-5-(4-(trifluoromethoxy)phenyl)-1,5-dihydro-4H-imidazo[4,5-c]pyridin-4-one as a pale beige-colored solid in 97.6% yield.

### Step 6: Synthesis of 1-methyl-7-(1-methyl-1H-pyrazol-4-yl)-5-(4-(trifluoromethoxy)phenyl)-1,5-dihydro-4H-imidazo[4,5-c]pyridin-4-one (Compound I)

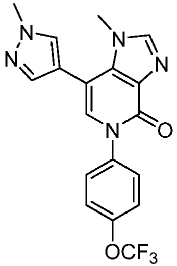

A reactor was charged with 7-bromo-1-methyl-5-(4-(trifluoromethoxy)phenyl)-1,5-dihydro-4*H*-imidazo[4,5-c]pyridin-4-one (1.0 mol equivalents), (1-methyl-1H-pyrazol-4-yl)boronic acid pinacol ester (1-methyl-4-(4,4,5,5-tetramethyl-1,3,2-dioxaborolan-2-yl)-1*H-*pyrazole, 1.6 mol equivalents), Pd[Ph₃]₄ (0.025 mol equivalents, and K₂CO₃ (2.0 mol equivalents), to which were added acetonitrile (10.0 volume equivalents) and water (3.0 volume equivalents). The reaction mixture was stirred for approximately 10-20 minutes at about 20-25 °C to form a suspension.

The mixture was heated to slight reflux, whereupon a biphasic, yellow solution formed. The mixture was stirred at slight reflux for at least 10 hours. The reaction mixture was cooled to between 30-50 °C, then passed through a particle filter. The filter was washed with acetonitrile (2.6 volume equivalents), the filtrates were combined, and the solution was concentrated to a final volume of approximately 120 mL (4.8 volume equivalents) under reduced pressure at below 60 °C.

To the resulting suspension was added water (1.9 volume equivalents), methanol (26 mL, 1.0 volume equivalents), and dichloromethane (14.8 volume equivalents). The mixture was warmed to about 30-35 °C and stirred until two clear layers were observed. The layers were allowed to separate without stirring at about 30-35 °C, and additional dichloromethane (3.7 volume equivalents) was added to the aqueous layer. The mixture was warmed to approximately 30-35 °C and stirred for about 5 minutes, and then the layers were allowed to separate at approximately 30-35 °C.

To the combined organic layers was added water (1.9 volume equivalents), and the mixture was warmed to approximately 30-35 °C and stirred for about 5 minutes. The layers were separated at approximately 30-35 °C. Charcoal was added to the combined organic layers and stirred for 30-60 minutes at approximately 30-35 °C. The charcoal was removed by filtration, and the filter was washed with dichloromethane (39 mL, 1.6 volume equivalents).

The solution was concentrated to approximately 4.0 volume equivalents at ambient pressure and at below 50 °C, then diluted with methanol (5.0 volume equivalents). The solution was again concentrated to approximately 4.0 volume equivalents at ambient pressure and below 60 °C, diluted with methanol (5.0 volume equivalents), and concentrated to a final volume of approximately 3.0 volume equivalents under reduced pressure below 60 °C.

To the resulting suspension was added methanol (2.9 volume equivalents), and the suspension was warmed to approximately 45-55 °C and stirred for about 1 hour. The suspension was cooled to approximately 0-5 °C within approximately 1 hour, stirred for 1 hour at approximately 0-5 °C, and then filtered. The filter cake was washed with cold methanol (pre-cooled to approximately 0-10 °C, 2.9 volume equivalents), and the product was dried under a stream of nitrogen and in vacuo at below 60 °C until the loss on drying was ≤ 1% by weight, giving Compound I (1-methyl-7-(1-methyl-1H-pyrazol-4-yl)-5-(4-(trifluoromethoxy)phenyl)-1,5-dihydro-4H-imidazo[4,5-c]pyridin-4-one) as a white solid in 88.5% yield.

### Step 7: Recrystallization of 1-methyl-7-(1-methyl-1H-pyrazol-4-yl)-5-(4-(trifluoromethoxy)phenyl)-1,5-dihydro-4H-imidazo[4,5-c]pyridin-4-one (Compound I)

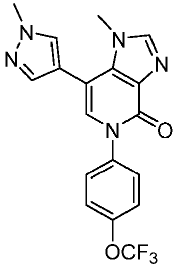

A reactor was charged with crude 1-methyl-7-(1-methyl-1*H*-pyrazol-4-yl)-5-(4-(trifluoromethoxy)phenyl)-1,5-dihydro-4*H*-imidazo[4,5-c]pyridin-4-one from step 6, and to this was added glacial acetic acid (1.5 volume equivalents). The suspension was warmed to approximately 50-60 °C and stirred until a clear solution was obtained, approximately 10-20 minutes. The warm solution was passed through a particle filter into a second reactor.

To this solution was added ethanol (10.0 volume equivalents) at approximately 45-55 °C over 2 hours. The suspension was stirred for approximately 30 minutes at approximately 45-55 °C, then cooled to approximately 0-5 °C over about 4 hours. The suspension was then stirred for approximately 4-16 hours at about 0-5 °C.

Next, the suspension was filtered and the filter cake was washed with cold isopropanol (4.2 volume equivalents) at approximately 0-20 °C. The product was dried under a nitrogen stream and in vacuo at below 60 °C until the loss on drying was ≤ 1% by weight, giving Compound I (1-methyl-7-(1-methyl-1H-pyrazol-4-yl)-5-(4-(trifluoromethoxy)phenyl)-1,5-dihydro-4*H*-imidazo[4,5-c]pyridin-4-one) as a white solid in 93.0% yield.

### Step 8: Synthesis of 1-methyl-7-(1-methyl-1H-pyrazol-4-yl)-5-(4-(trifluoromethoxy)phenyl)-1,5-dihydro-4H-imidazo[4,5-c]pyridin-4-one, mono-tosylate salt (Compound I mono-tosylate salt)

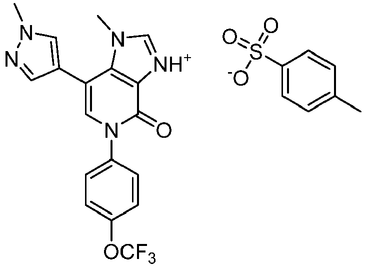

A reactor was charged with Compound I (1-methyl-7-(1-methyl-1H-pyrazol-4-yl)-5-(4-(trifluoromethoxy)phenyl)-1,5-dihydro-4H-imidazo[4,5-c]pyridin-4-one, 1.00 mol equivalent), para-toluenesulfonic acid monohydrate (1.05 mol equivalents), acetone (6.75 volume equivalents), and water (0.75 volume equivalents). The mixture was stirred at 15-25 °C until a clear solution formed, and then this solution was filtered through a particle filter into a second reactor.

The filter was washed with acetone (2.5 volume equivalents), and to the combined filtrates was added MTBE (7.5 volume equivalents) at 15-25 °C and Compound I mono-tosylate seeding crystals (0.001 mol equivalents).

The resulting suspension was stirred at 15-25 °C for approximately 30-60 minutes, and MTBE was added (22.5 volume equivalents) at 15-25 °C during a period of approximately 30 minutes. Stirring was continued at 15-25 °C for approximately 30-60 minutes, and then the suspension was filtered. The filter was washed with MTBE (2.5 volume equivalents), and the material was dried in vacuo at below 55 °C to give Compound I mono-tosylate salt (1-methyl-7-(1-methyl-1*H*-pyrazol-4-yl)-5-(4-(trifluoromethoxy)phenyl)-1,5-dihydro-4*H*-imidazo[4,5-c]pyridin-4-one, mono-tosylate salt) as a white, crystalline solid in 93% yield.

### Example 14 - Compound I Free Base Nanosuspension Tablet

A nanosuspension of Compound I was integrated into a solid dosage form through a fluid bed granulation process. To support pharmacokinetic studies, two dose strengths at 25 and 200 mg Compound I with corresponding placebos were produced.

Purified water was heated to 30-40 °C, to which were added HPMC and polysorbate 80 under medium mixing to form a suspension vehicle consisting of water (98.6% w/w), polysorbate 80 (0.56% w/w), and HPMC (0.84% w/w). An amount of Compound I was added to the suspension vehicle, and the mixture was transferred to a wet mill charged with conditioned ZrO₂ grinding media. The mixture was milled under nitrogen for 405±5 minutes to produce a nanosuspension including Compound I (10.0% w/w), suspension vehicle (71.5% w/w), and additional deionized water (18.5% w/w).

Next, the nanosuspension was granulated. Mannitol was added to the nanosuspension prepared in the previous step, and the suspension was mixed for 15 minutes to ensure dissolution. A fluid bed was charged with microcrystalline cellulose, and the nanosuspension mixture was sprayed thereupon. Once the nanosuspension was completely dispersed over the cellulose, spraying was stopped and the bed temperature was raised above 40 °C to facilitate drying. This blend was milled to produce granules having a particle size D₅₀ of 135 µm, which were pressed into a tablet.

### Example 15 - Jet-milled Compound I Free Base Tablet

Jet-milled Compound I, microcrystalline cellulose, lactose and croscarmellose sodium were sieved through a 20 mesh screen. Magnesium stearate was sieved through a 30 mesh screen. The sieved components were mixed and blended for 5 minutes. Intragranular magnesium stearate was added and blended for 3 minutes. Slugs were prepared using a press, the slugs were ground by mortar and pestle, and the ground powder was sieved through a 20 mesh screen. Extragranular croscarmellose sodium was added and blended for 5 minutes. Extragranular magnesium stearate was added and blended for 3 minutes to obtain the final blend. The final compositions for two prepared batches are shown in Tables 7 and 8, below.

**Table 7**

| **Component** | | **% w/w** | **Mass/Tablet (mg)** | **Theoretical Amount (g)** | **Actual Amount (g)** |
|---|---|---|---|---|---|
| Compound I | | 25.00 | 200 | 10 | 10.0011 |
| Microcrystalline cellulose | | 52.50 | 420 | 21 | 21.0011 |
| Lactose | | 17.5 | 140 | 7 | 7.001 |
| Croscarmellose | Intra | 2.00 | 16 | 0.8 | 0.8017 |
| sodium | Extra | 2.00 | 16 | 0.8 | 0.79805 |
| Magnesium stearate | Intra | 0.50 | 4 | 0.2 | 0.20099 |
| | Extra | 0.50 | 4 | 0.2 | 0.1997 |
| Total | | 100 | 800 | 40 | 40.00364 |

**Table 8**

| **Component** | | **% w/w** | **Mass/Tablet (mg)** | **Theoretical Amount (g)** | **Actual Amount (g)** |
|---|---|---|---|---|---|
| Compound I | | 25.00 | 200 | 10 | 10.0023 |
| Microcrystalline cellulose | | 52.50 | 420 | 21 | 21.0000 |
| Lactose | | 17.5 | 140 | 7 | 7.0001 |
| Croscarmellose sodium | Intra | 2.00 | 16 | 0.8 | 0.8002 |
| | Extra | 2.00 | 16 | 0.8 | 0.7941 |
| Magnesium stearate | Intra | 0.50 | 4 | 0.2 | 0.2001 |
| | Extra | 0.50 | 4 | 0.2 | 0.1986 |
| Total | | 100 | 800 | 40 | 39.995 |

Tablets were compressed with an oval shape tooling. Press pressure was 2.0 ton, and dwell time was 30 seconds. Tablet weight, thickness and hardness were 796 - 809 mg, 5.48 - 5.56 mm and 162 - 201 N, respectively.

### Example 16 - Compound I Mono-Tosylate Tablet

Compound I mono-tosylate salt, microcrystalline cellulose, and lactose were sieved through a 20 mesh screen. Magnesium stearate was sieved through a 30 mesh screen. The microcrystalline cellulose, lactose, and intragranular croscarmellose sodium were blended with for 5 minutes, intragranular magnesium stearate was added, and the mixture was blended for 3 minutes. Slugs were prepared, then ground, and the resulting powder was sieved through a 20 mesh screen. Extragranular croscarmellose sodium and HPMC were added and the mixture was blended for 5 minutes to produce the final blend. The final composition is shown in Table 9, below.

**Table 9**

| **Component** | | **% w/w** | **Mass/Tablet (mg)** | **Mass in Blend (g)** | **Actual Weight (g)** |
|---|---|---|---|---|---|
| Compound I mono-tosylate | | 36.06 | 288.5 | 7.9332 | 7.9335 |
| Microcrystalline cellulose | | 43.84 | 350.7 | 9.6448 | 9.6440 |
| Lactose | | 14.60 | 116..8 | 3.212 | 3.2130 |
| Croscarmellose sodium | Intra | 2 | 16.0 | 0.44 | 0.4406 |
| | Extra | 2 | 16.0 | 0.44 | 0.4322 |
| Magnesium stearate | Intra | 0.5 | 4.0 | 0.11 | 0.1101 |
| | Extra | 0.5 | 4.0 | 0.11 | 0.1081 |
| HPMC | | 0.5 | 4.0 | 0.11 | 0.1072 |
| Total | | 100 | 800 | 22 | 21.9887 |

Tablets were manufactured from the final blend by an oval shape tooling. Tablet weight and thickness were controlled to within 797-807 mg and 5.56-5.70 mm, respectively. Tablet hardness was 101-197 N.

### Example 17 - Compound I Di-Tosylate Tablet

Compound I di-tosylate salt was sieved through a 35 mesh screen, and all excipients were sieved through a 20 or 30 mesh screen. Compound I di-tosylate salt was mixed with microcrystalline cellulose, lactose, and intragranular croscarmellose sodium, then blended for 10 minutes. Magnesium stearate was added and the mixture was blended for another 2 minutes. Slugs were prepared by pressing at 1.6 ton pressure, each containing 4 g powder. The slugs were ground by mortar and pestle, and then sieved through a 20 mesh screen. Extragranular croscarmellose sodium and HPMC (to inhibit salt precipitation) were added to the ground powder and blended for 4 minutes. Extragranular magnesium stearate was added and blended for 1 minute to obtain the final blend. The final composition is shown in Table 10, below.

**Table 10**

| **Component** | | **% w/w** | **Mass/Tablet (mg)** | **Mass in Blend (g)** | **Actual Weight (g)** |
|---|---|---|---|---|---|
| Compound I di-tosylate | | 36.06 | 288.5 | 7.9332 | 7.9335 |
| Microcrystalline cellulose | | 43.84 | 350.7 | 9.6448 | 9.6440 |
| Lactose | | 14.60 | 116..8 | 3.212 | 3.2130 |
| Croscarmellose sodium | Intra | 2 | 16.0 | 0.44 | 0.4406 |
| | Extra | 2 | 16.0 | 0.44 | 0.4322 |
| Magnesium stearate | Intra | 0.5 | 4.0 | 0.11 | 0.1101 |
| | Extra | 0.5 | 4.0 | 0.11 | 0.1081 |
| HPMC | | 0.5 | 4.0 | 0.11 | 0.1072 |
| Total | | 100 | 800 | 22 | 21.9887 |

Tablets were manufactured from the final blend by 12 mm round flat-faced tooling. The tablet size was targeted at 800 mg, with compression at 1.0 ton pressure. The tablet hardness was determined to be around 180 N. Tablet weight ranged from 796 to 803 mg, and thickness from 4.85 to 4.98 mm.

### Example 18 - Compound I Free Base Amorphous Solid Dispersion Tablet

An amorphous solid dispersion (ASD) of Compound I was prepared at 50% drug loading as follows: Compound I free base and HPMC acetate succinate (HPMCAS-MF) were dissolved in methanol/dichloromethane (v/v= 1:1). The solution was then spray dried via a mini spray dryer at inlet temperature 75 °C, and further dried in a vacuum oven at 25 °C overnight.

The ASD was sieved through a 20 mesh screen, and all excipients were sieved through a 20 or 30 mesh screen. The sieved ASD was mixed with microcrystalline cellulose, lactose, and intragranular croscarmellose sodium, then blended for 5 minutes. Intragranular magnesium stearate was then added, and the mixture was blended for 3 minutes. The preblend was slugged, and the slugs were then ground with mortar and the resulting powder sieved through a 20 mesh screen. Extragranular croscarmellose sodium was added and blended with ground powder for 5 min. Extragranular magnesium stearate was then added, and the mixture blended for 3 minutes to obtain the final blend. The final compositions for two prepared batches are shown in Tables 11 and 12, below.

**Table 11**

| **Component** | | **% w/w** | **Mass/Tablet (mg)** | **Theoretical Amount (g)** | **Actual Amount (g)** |
|---|---|---|---|---|---|
| Compound I ASD | | 50.00 | 400 | 35 | 35.00000 |
| Microcrystalline cellulose | | 22.50 | 180 | 15.75 | 15.75000 |
| Lactose | | 22.50 | 180 | 15.75 | 15.75020 |
| Croscarmellose sodium | Intra | 2.00 | 16 | 1.4 | 1.40020 |
| | Extra | 2.00 | 16 | 1.4 | 1.38979 |
| Magnesium stearate | Intra | 0.50 | 4 | 0.35 | 0.35000 |
| | Extra | 0.5 | 4 | 0.35 | 0.34746 |
| Total | | 100 | 800 | 70 | 69.98765 |

**Table 12**

| **Component** | | **% w/w** | **Mass/Tablet (mg)** | **Theoretical Amount (g)** | **Actual Amount (g)** |
|---|---|---|---|---|---|
| Compound I ASD | | 50.00 | 400 | 9 | 9.0003 |
| Microcrystalline cellulose | | 21.50 | 172 | 3.87 | 3.8701 |
| Lactose | | 21.50 | 172 | 3.87 | 3.8702 |
| Croscarmellose sodium | Intra | 2.50 | 20 | 0.45 | 0.4500 |
| | Extra | 3.50 | 28 | 0.63 | 0.6208 |
| Magnesium stearate | Intra | 0.50 | 4 | 0.09 | 0.0902 |
| | Extra | 0.5 | 4 | 0.09 | 0.0888 |
| Total | | 100 | 800 | 18 | 17.9904 |

Tablets were compressed with an 800 mg oval shape tooling using a press compression pressure of 2 ton. The hardness of the produced tablets ranged from 140 to 190 N. Weight and thickness of the tablets ranged from 798 to 802 mg and from 5.83 to 5.91 mm, respectively.

### Examples 19-21 - Canine Pharmacokinetics Assays

Pharmacokinetics parameters of formulations of Compound I and its salts in dogs were determined by orally administering a test formulation tablet at 200 mg of active (Compound I or salt) per dog.

Blood samples were taken at various times after administration, plasma prepared, and submitted to analysis for parent drug using a qualified LC-MS-MS assay. Pharmacokinetics parameters derived from the plasma analytical data were determined using non-compartmental analysis.

### Example 19 - Crossover Pharmacokinetics Study of Four Oral Formulations of Compound I in Dogs

Pharmacokinetics of Compound I was determined in pentagastrin-pretreated male beagle dogs following a single oral (PO) administration at 200 mg/tablet/dog in a 4-phase crossover study with a washout period of at least 7 days in between each phase. Five male non-naive purebred beagle dogs were used in each phase of study. All dogs were pretreated with pentagastrin (6 µg/kg intramuscular administrations) approximately 30 minutes before administration of test article.

Four tablet formulations of test article were assessed, each containing 200 mg test article (free base equivalent). Animals were fasted overnight prior to PO dosing through approximately 4 hours postdose. The tablet dose was administered orally, followed by approximately 10 mL of RO (reverse osmosis) water. Phase 1 animals were given tablets containing Compound I in nanosuspension formulation as described above, phase 2 animals were given tablets containing Compound I in jet-milled form as described above, and phase 4 animals received tablets containing Compound I mono-tosylate salt as described above. In phase 3, tablets including an amorphous solid dispersion as described above were crushed and suspended in water, then dosed to the animals. Each animal received one triturated tablet, which was administered orally by gavage as an aqueous suspension formulation. Blood samples were collected predose and at 0.25, 0.5 1, 2, 4, 8, 24, 36 and 48 hours after the oral dose. Plasma was harvested, and the concentration of Compound I in each sample was determined by a non-validated LC/MS/MS assay. The assay limit of quantitation was 1.02 to 5.1 ng/mL for Compound I.

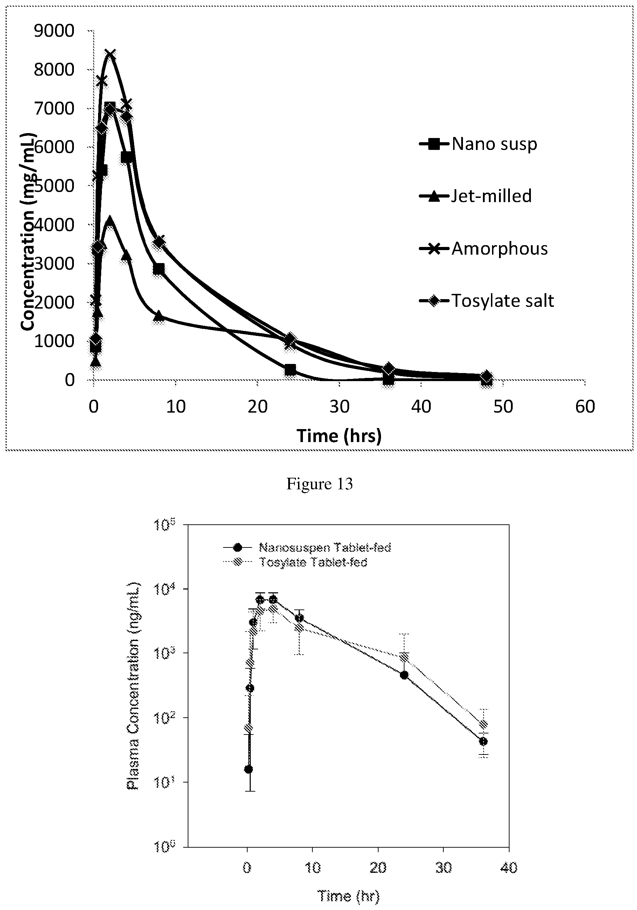

Mean Compound I concentrations measured in plasma were used to construct semi-logarithmic plasma concentration-time curves (Figure 13). Pharmacokinetic analysis was performed using a non-compartmental method.

The PK parameters are presented in Table 13. Mean plasma concentration-time profiles of Compound I following PO doses are presented in Figure 13. Individual and mean plasma concentration from each phase are presented in Tables 14-17. AUC₀₋ₗₐₛₜ, Cₘₐₓ and tₘₐₓ were 56300 ng-hr/mL, 7020 ng/mL, and 2.00 hr, respectively for phase 1 (nanosuspension), 46200 ng-hr/mL, 4290 ng/mL, and 6.40 hr, respectively for phase 2 (jet-milled), 82900 ng-hr/mL, 8460 ng/mL, 2.40 hr, respectively for phase 3 (amorphous solid dispersion), and 78500 ng-hr/mL, 8100 ng/mL, 1.80 hr, respectively for phase 4 (mono-tosylate salt). Based on the mean PK data, Compound I exposures (AUG and Cₘₐₓ) in dogs receiving the amorphous tablet (crushed in water, phase 3) and mono-tosylate salt tablet (phase 4) are comparable, and the exposures from these two tablet formulations are higher (within two fold) compared with the tablet in phases 1 and 2 (nanosuspension and jet-milled formulations).

**Table 13**

| Tablet Formulation | Subject | AUC₀₋ₗₐₛₜ (ng·hr/mL) | AUC₀₋ᵣₜ (ng. hr/mL) | Cₘₐₓ (ng/mL) | tₘₐₓ (hr) |
|---|---|---|---|---|---|
| Nanosuspension | 1 | 69000 | 69100 | 8130 | 2.00 |
| | 2 | 52800 | 52900 | 6310 | 2.00 |
| | 3 | 37700 | 37700 | 5440 | 2.00 |
| | 4 | 99800 | 99800 | 11900 | 2.00 |
| | 5 | 22100 | 22100 | 3350 | 2.00 |
| | Mean | 56300 | 56300 | 7020 | 2.00 |
| | SD | 29900 | 29900 | 3210 | 0.00 |
| Jet-milled | 1 | 24900 | 25000 | 3190 | 2.00 |
| | 2 | 46300 | 46400 | 4940 | 2.00 |
| | 3 | 42900 | 43000 | 6570 | 2.00 |
| | 4 | 101000 | N/A | 4650 | 24.0 |
| | 5 | 15400 | 15700 | 2080 | 2.00 |
| | Mean | 46200 | 32500 | 4290 | 6.40 |
| | SD | 33400 | 14700 | 1720 | 9.84 |
| Amorphous Solid Dispersion | 1 | 110000 | 110000 | 12900 | 2.00 |
| | 2 | 59400 | 59500 | 7640 | 2.00 |
| | 3 | 71200 | N/A | 8680 | 4.00 |
| | 4 | 136000 | 137000 | 8390 | 2.00 |

| Tablet Formulation | Subject | AUC₀₋ₗₐₛₜ (ng·hr/mL) | AUC₀₋ᵣₜ (ng·hr/mL) | Cₘₐₓ (ng/mL) | tₘₐₓ (hr) |
|---|---|---|---|---|---|
| | 5 | 37500 | 37600 | 4670 | 2.00 |
| | Mean | 82900 | 86100 | 8460 | 2.40 |
| | SD | 39800 | 45700 | 2960 | 0.894 |
| Mono-Tosylate Salt | 1 | 76500 | 76600 | 9450 | 1.00 |
| | 2 | 116000 | 116000 | 12900 | 4.00 |
| | 3 | 44300 | 44400 | 7310 | 1.00 |
| | 4 | 122000 | 123000 | 5450 | 2.00 |
| | 5 | 34200 | 34300 | 5360 | 1.00 |
| | Mean | 78500 | 78900 | 8100 | 1.80 |
| | SD | 40000 | 40400 | 3180 | 1.30 |

**Table 14**

| Time (hours) | 1 | 2 | 3 | 4 | 5 | Mean | SD |
|---|---|---|---|---|---|---|---|
| Pre-dose | BLQ | BLQ | BLQ | BLQ | BLQ | BLQ | N/A |
| 0.25 | 364 | 1140 | 1430 | 1080 | 259 | 854 | 514 |
| 0.5 | 3270 | 4030 | 3650 | 4100 | 1830 | 3380 | 924 |
| 1 | 6460 | 5310 | 4750 | 7390 | 3100 | 5400 | 1640 |
| 2 | 8130 | 6310 | 5440 | 11900 | 3350 | 7020 | 3210 |
| 4 | 6580 | 5200 | 4570 | 9960 | 2370 | 5740 | 2810 |
| 8 | 3700 | 2460 | 2040 | 5110 | 986 | 2860 | 1590 |
| 24 | 357 | 309 | 21.2 | 562 | 53.0 | 261 | 225 |
| 36 | 12.1 | 12.5 | 2.37 | 41.4 | 3.15 | 14.3 | 15.9 |
| 48 | 2.84 | 2.49 | 1.04 | 10.1 | 1.54 | 3.59 | 3.68 |

**Table 15**

| Time (hours) | 1 | 2 | 3 | 4 | 5 | Mean | SD |
|---|---|---|---|---|---|---|---|
| Pre-dose | BLQ | BLQ | BLQ | BLQ | BLQ | BLQ | N/A |
| 0.25 | 596 | 702 | 124 | 798 | 219 | 488 | 300 |
| 0.5 | 1830 | 2210 | 1420 | 2080 | 1280 | 1760 | 405 |
| 1 | 3040 | 4180 | 4510 | 3930 | 1920 | 3510 | 1050 |
| 2 | 3190 | 4940 | 6570 | 3740 | 2080 | 4100 | 1720 |
| 4 | 2070 | 4410 | 5230 | 2750 | 1660 | 3220 | 1540 |
| 8 | 1330 | 2360 | 2430 | 1510 | 673 | 1660 | 742 |
| 24 | 114 | 310 | 38.8 | 4650 | 45.1 | 1030 | 2030 |
| 36 | 7.25 | 12.0 | BLQ | 891 | 21.0 | 233 | 439 |
| 48 | BLQ | BLQ | BLQ | 105 | 13.2 | 59.1 | N/A |

**Table 16**

| Time (hours) | 1 | 2 | 3 | 4 | 5 | Mean | SD |
|---|---|---|---|---|---|---|---|
| Pre-dose | BLQ | BLQ | BLQ | BLQ | BLQ | BLQ | N/A |
| 0.25 | 3400 | 1730 | 2280 | 2070 | 846 | 2070 | 925 |
| 0.5 | 7520 | 5330 | 5310 | 5370 | 2780 | 5260 | 1680 |
| 1 | 11300 | 6570 | 8180 | 8350 | 4100 | 7710 | 2660 |
| 2 | 12900 | 7640 | 8360 | 8390 | 4670 | 8390 | 2950 |
| 4 | 9290 | 6170 | 8680 | 7230 | 4200 | 7110 | 2040 |
| 8 | 5330 | 2810 | 3800 | 4330 | 1740 | 3600 | 1380 |
| 24 | 777 | 236 | 138 | 3340 | 123 | 923 | 1380 |
| 36 | 32.5 | 7.55 | BLQ | 683 | 29.2 | 188 | 330 |
| 48 | 5.46 | BLQ | BLQ | 129 | 12.5 | 48.9 | 69.2 |

**Table 17**

| Time (hours) | 1 | 2 | 3 | 4 | 5 | Mean | SD |
|---|---|---|---|---|---|---|---|
| Pre-dose | BLQ | BLQ | BLQ | BLQ | BLQ | BLQ | N/A |
| 0.25 | 309 | 1500 | 2900 | 39.7 | 571 | 1060 | 1160 |
| 0.5 | 3650 | 3910 | 5440 | 726 | 3520 | 3450 | 1700 |
| 1 | 9450 | 5470 | 7310 | 4880 | 5360 | 6490 | 1900 |
| 2 | 9370 | 10200 | 5680 | 5450 | 4130 | 6960 | 2650 |
| 4 | 8040 | 12900 | 4590 | 4840 | 3480 | 6780 | 3840 |
| 8 | 3650 | 6910 | 2500 | 3060 | 1630 | 3550 | 2020 |
| 24 | 352 | 597 | BLQ | BLQ | BLQ | 1070 | 1810 |
| 36 | 11.0 | 17.8 | BLQ | 842 | BLQ | 290 | 478 |
| 48 | BLQ | BLQ | BLQ | 200 | 10.1 | 105 | N/A |

### Example 20 - Crossover Pharmacokinetics Study of Two Oral Formulations of Compound I in Dogs under Fed Conditions

Pharmacokinetics of Compound I were determined in pentagastrin-pretreated male beagle dogs following a single oral (PO) administration at 200 mg/tablet/dog in a 2-phase crossover study with a washout period of at least 7 days in between each phase. Six male, non-naive, purebred beagle dogs were used in each phase of the 2-phase crossover study. All dogs were pretreated with pentagastrin (6 µg/kg intramuscular administrations) approximately 30 minutes before test article administration.

Two tablet formulations (nanosuspension and mono-tosylate salt, as described above) of test article were assessed. Phase 1 animals were given tablets containing Compound I nanosuspension. Phase 2 animals were given tablets containing Compound I mono-tosylate salt. Animals in both phases were fasted overnight through approximately 4 hours postdose, and received a 100-mL volume of homogenized Food and Drug Administration (FDA) high fat diet by oral gavage at approximately 30 minutes prior to test article administration, followed by a 7 to 10-mL flush with water. Blood samples were collected pre-dose and at 0.25. 0.5, 1. 2. 4. 8, 24, 36, and 48 hours after the oral dose. Plasma was harvested, and the concentration of Compound I in each plasma sample was determined by an LC/MS/MS assay. The assay lower limit of quantitation was 20 ng/mL for Compound I.

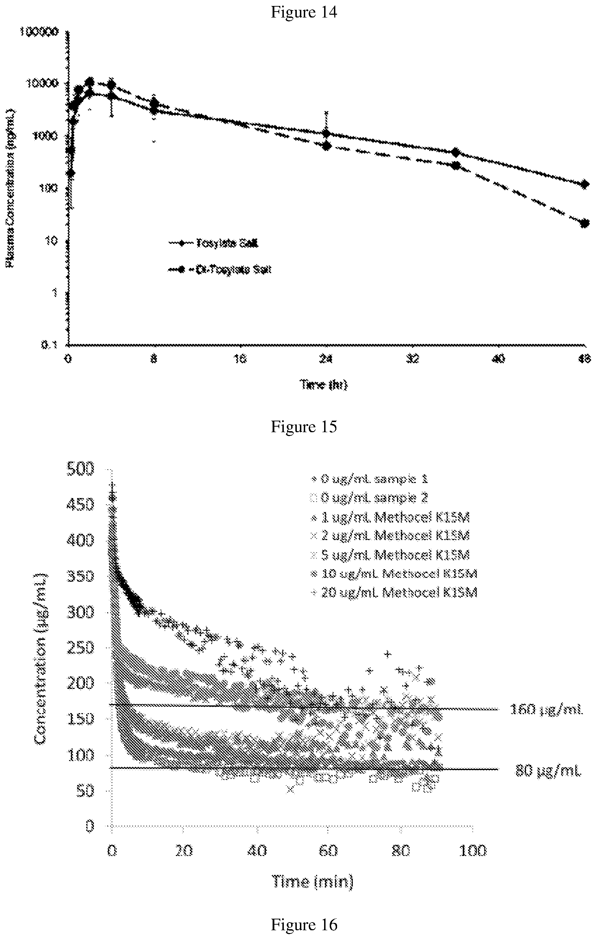

Mean Compound I concentrations measured in plasma were used to construct semi-logarithmic plasma concentration-time curves (Figure 14). Pharmacokinetic analysis was performed using non-compartmental methods.

The PK parameters of Compound I are presented in Table 18. Mean plasma concentration-time profiles of Compound I following PO doses of Compound I in nanosuspension and mono-tosylate formulation are presented in Figure 14. Individual and mean plasma concentration from each phase are presented in Tables 19 and 20. AUC₀₋ₗₐₛₜ, Cₘₐₓ, and tₘₐₓ were 63100 ng-hr/mL, 7210 ng/mL, and 3.33 hr, respectively, for phase 1 (nanosuspension), and 48800 ng.hr/mL, 5430 ng/mL, and 3.00 hr, respectively, for phase 2 (mono-tosylate salt). Based on the mean PK data, in the fed state, the Compound I exposure in dogs with dose of tablet in nanosuspension formulation was slightly higher than that with the mono-tosylate salt formulation (i.e., the mono-tosylate salt AUC and Cₘₐₓ are about 75-85% of the nanosuspension under fed conditions). This contrasts to performance under fasted conditions, wherein the mono-tosylate salt had a higher AUC and Cₘₐₓ compared to the nanosuspension (i.e., 115-140% of the nanosuspension).

**Table 18**

| Tablet Formulation | Subject | AUC₀₋ₗₐₛₜ (ng·hr/mL) | AUC₀₋ᵣₜ (ng·hr/mL) | Cₘₐₓ (ng/mL) | tₘₐₓ (hr) |
|---|---|---|---|---|---|
| Nanosuspension | 1 | 55200 | 55600 | 5100 | 4.00 |
| | 2 | 59900 | N/A | 6700 | 4.00 |
| | 3 | 77100 | 77500 | 7620 | 2.00 |
| | 4 | 30200 | N/A | 4800 | 4.00 |
| | 5 | 71200 | N/A | 9770 | 4.00 |
| | 6 | 84900 | 85000 | 9250 | 2.00 |
| | Mean | 63100 | 72700 | 7210 | 3.33 |
| | SD | 19500 | 15300 | 2070 | 1.03 |
| Mono-Tosylate salt | 1 | 59000 | 59700 | 5340 | 4.00 |
| | 2 | 75300 | NA | 8590 | 4.00 |
| | 3 | 68500 | 70100 | 5160 | 2.00 |
| | 4 | 26200 | 26200 | 4930 | 2.00 |
| | 5 | 16000 | N/A | 3490 | 4.00 |
| | 6 | 47500 | 47700 | 5080 | 2.00 |
| | Mean | 48800 | 50900 | 5430 | 3.00 |
| | SD | 23600 | 18800 | 1680 | 1.10 |

**Table 19**

| Time (hours) | 1 | 2 | 3 | 4 | 5 | 6 | Mean | SD |
|---|---|---|---|---|---|---|---|---|
| Pre-dose | BLQ | BLQ | BLQ | BLQ | BLQ | BLQ | BLQ | N/A |
| 0.25 | BLQ | BLQ | BLQ | BLQ | 95.5 | BLQ | 15.9 | 39.0 |
| 0.5 | 43.0 | 41.1 | 487 | 26.2 | 644 | 517 | 293 | 286 |
| 1 | 2050 | 1740 | 6240 | 1030 | 4090 | 3130 | 3040 | 1900 |
| 2 | 4920 | 6660 | 7620 | 4350 | 8200 | 9250 | 6830 | 1910 |
| 4 | 5100 | 6700 | 6640 | 4800 | 9770 | 8270 | 6880 | 1890 |
| 8 | 3470 | 4310 | 2590 | 1650 | 4560 | 4840 | 3570 | 1250 |
| 24 | 375 | 163 | 1500 | 25.8 | 95.1 | 591 | 459 | 552 |
| 36 | 57.4 | BLQ | 45.6 | BLQ | BLQ | 26.2 | 43.1 | 15.7 |
| 48 | BLQ | BLQ | BLQ | BLQ | BLQ | BLQ | BLQ | N/A |

**Table 20**

| Time (hours) | 1 | 2 | 3 | 4 | 5 | 6 | Mean | SD |
|---|---|---|---|---|---|---|---|---|
| Pre-dose | BLQ | BLQ | BLQ | BLQ | BLQ | BLQ | BLQ | N/A |
| 0.25 | BLQ | 381 | 41.0 | BLQ | BLQ | BLQ | 70.3 | 153 |
| 0.5 | 49.2 | 3630 | 532 | 57.7 | 31.8 | BLQ | 717 | 1440 |
| 1 | 107 | 6070 | 2340 | 3330 | 1200 | 97.9 | 2190 | 2280 |
| 2 | 2010 | 8270 | 5160 | 4930 | 2020 | 5080 | 4580 | 2350 |
| 4 | 5340 | 8590 | 4020 | 3770 | 3490 | 4530 | 4960 | 1890 |
| 8 | 3240 | 4900 | 1480 | 1020 | 1200 | 2920 | 2460 | 1510 |
| 24 | 913 | 156 | 2800 | 20.8 | BLQ | 342 | 847 | 1140 |
| 36 | 88.7 | BLQ | 130 | BLQ | BLQ | 20.5 | 79.7 | 55.2 |
| 48 | BLQ | BLQ | BLQ | BLQ | BLQ | BLQ | BLQ | N/A |

### Example 21 - Crossover Pharmacokinetics Study of Oral Formulations of Compound I Mono-Tosylate and Di-Tosylate Salts in Dogs

Pharmacokinetics of Compound I were determined in pentagastrin-pretreated male beagle dogs following a single oral (PO) administration at 200 mg/tablet per dog in a 2-phase crossover designed study, with a washout period of at least 7 days in between each phase. Five male, non-naive, purebred beagle dogs were used in each phase of the 2-phase crossover study. All dogs were pretreated with pentagastrin (6 µg/kg intramuscular administrations)approximately 30 minutes before test article administration.

Two tablet formulations (mono-tosylate and di-tosylate, as described above) of test article were assessed. Animals were fasted overnight prior to PO dosing through approximately 4 hours post-dose. The tablet was administered orally, followed by approximately 10 mL of reverse osmosis water. Phase 1 animals were given tablets containing Compound I mono-tosylate, and phase 2 animals were given tablets containing Compound I di-tosylate salt. Blood samples were collected pre-dose and at 0.25, 0.5. 1. 2, 4, 8, 24, 36, and 48 hours after the oral dose. Plasma was harvested, and the concentration of Compound I in each plasma sample was determined by an LC/MS/MS assay. The assay lower limit of quantitation was 20 ng/mL for Compound I. Mean Compound I concentrations measured in plasma were used to construct semi-logarithmic plasma concentration-time curves (Figure 1). Pharmacokinetic analysis was performed using non-compartmental methods.

The PK parameters of Compound I are presented in Table 21. Mean plasma concentration-time profiles of Compound I following PO doses of two formulations are presented in Figure 15. Individual and mean plasma concentrations from each phase are presented in Tables 22 and 23. AUC₀₋ₗₐₛₜ, Cₘₐₓ, and tₘₐₓ were 70200 ng-hr/mL, 6480 ng/mL, and 2.00 hr, respectively, for phase 1 (mono-tosylate salt), and 88000 ng-hr/mL, 10400 ng/mL, and 2.00 hr, respectively, for phase 2 (di-tosylate salt). Based on the mean PK data, Compound I exposure in dogs per dose of the di-tosylate salt formulation is slightly higher than that of the mono-tosylate salt formulation.

**Table 21**

| Tablet Formulation | Subject | AUC₀₋ₗₐₛₜ (ng·hr/mL) | AUC₀₋ᵣₜ (ng·hr/mL) | Cₘₐₓ (ng/mL) | tₘₐₓ (hr) |
|---|---|---|---|---|---|
| Mono-tosylate | 1 | 26900 | 27400 | 3570 | 2.00 |
| | 2 | 122000 | 123000 | 5940 | 2.00 |
| | 3 | 77900 | 79400 | 9410 | 2.00 |
| | 4 | 110000 | 110000 | 10500 | 2.00 |
| | 5 | 14000 | 14100 | 2960 | 2.00 |
| | Mean | 70200 | 70900 | 6480 | 2.00 |
| | SD | 48500 | 48700 | 3400 | 0.00 |
| Di-tosylate | 1 | 62700 | 64100 | 8810 | 2.00 |
| | 2 | 129000 | 129000 | 11200 | 2.00 |
| | 3 | 113000 | 116000 | 13400 | 2.00 |
| | 4 | 9680 | 9890 | 1600 | 1.00 |
| | 5 | 47200 | 47300 | 8110 | 2.00 |
| | Mean | 88000 | 89100 | 10400 | 2.00 |
| | SD | 39200 | 39500 | 2410 | 0.00 |

**Table 22**

| Time (hours) | 1 | 2 | 3 | 4 | 5 | Mean | SD |
|---|---|---|---|---|---|---|---|
| Pre-dose | BLQ | BLQ | BLQ | BLQ | BLQ | BLQ | N/A |
| 0.25 | 179 | 452 | 100 | 74.0 | 161 | 193 | 151 |
| 0.5 | 1180 | 3040 | 1320 | 3170 | 850 | 1910 | 1100 |
| 1 | 2960 | 5270 | 5400 | 7730 | 2100 | 4690 | 2230 |
| 2 | 3570 | 5940 | 9410 | 10500 | 2960 | 6480 | 3400 |
| 4 | 3550 | 5080 | 7990 | 10200 | 1850 | 5730 | 3360 |
| 8 | 1140 | 3110 | 5060 | 5380 | 378 | 3010 | 2250 |
| 24 | 95.5 | 4080 | 252 | 1050 | 25.7 | 1100 | 1710 |
| 36 | BLQ | 897 | BLQ | 74.4 | BLQ | 486 | N/A |
| 48 | BLQ | BLQ | BLQ | BLQ | BLQ | BLQ | N/A |

**Table 23**

| Time (hours) | 1 | 2 | 3 | 4 | 5 | Mean | SD |
|---|---|---|---|---|---|---|---|
| Pre-dose | BLQ | BLQ | BLQ | BLQ | BLQ | BLQ | N/A |
| 0.25 | 1020 | 146 | 309 | 29.1 | 670 | 536 | 390 |
| 0.5 | 7120 | 1820 | 3500 | 636 | 2610 | 3760 | 2340 |
| 1 | 8040 | 6510 | 9340 | 1600 | 6140 | 7510 | 1470 |
| 2 | 8810 | 11200 | 13400 | 1580 | 8110 | 10400 | 2410 |
| 4 | 6820 | 10500 | 13100 | 1040 | 6440 | 9220 | 3170 |
| 8 | 2700 | 5620 | 5860 | 368 | 2040 | 4060 | 1970 |
| 24 | 231 | 1800 | 530 | 35.0 | 30.0 | 648 | 795 |
| 36 | BLQ | 273 | BLQ | BLQ | BLQ | 273 | N/A |
| 48 | BLQ | 21.5 | BLQ | BLQ | BLQ | 21.5 | N/A |

### Example 23 - Effect of polymer precipitation inhibitor

The inclusion of HPMC in the tablet of Example 16 was to maintain the supersaturation of Compound I API after its mono-tosylate salt dissolves. This effect is demonstrated in Figure 16, which shows the results of dissolution and precipitation of Compound I mono-tosylate salt when placed into pH 6.5 phosphate buffer at 37 °C containing HPMC at different concentrations. In the absence of HPMC in the formulation, after the mono-tosylate salt dissolved into pH 6.5 phosphate buffer at 37 °C, the API concentration decreased to 80 µg/mL in 10 minutes, which is approximately the solubility of Compound I free base form A. At low HPMC concentration (1 or 2 µg/mL) in the buffered solution, the polymer did not substantially maintain Compound I supersaturation. However, as the HPMC concentration increased to 5 µg/mL, the polymer was able to maintain Compound I concentration at 160 µg/mL, twice as much the free base Form A solubility. The HPMC concentration in this test of 5 µg/mL is equivalent to its 0.50% weight percentage in the tablet composition.

The foregoing description is given for clearness of understanding only, and no unnecessary limitations should be understood therefrom, as modifications within the scope of the invention may be apparent to those having ordinary skill in the art.

Throughout this specification and the claims which follow, unless the context requires otherwise, the word "comprise" and variations such as "comprises" and "comprising" will be understood to imply the inclusion of a stated integer or step (e.g. agents, elements, steps, or other features) or group of integers or steps but not the exclusion of any other integer or step or group of integers or steps.

Throughout the specification, where compositions are described as including components or materials, it is contemplated that the compositions can also consist essentially of, or consist of, any combination of the recited components or materials, unless described otherwise. Likewise, where methods are disclosed as including particular steps, it is contemplated that the methods can also consist essentially of, or consist of, any combination of the recited steps, unless described otherwise. The invention illustratively disclosed herein suitably may be practiced in the absence of any element or step which is not specifically disclosed herein.

The practice of a method disclosed herein, and individual steps thereof, can be performed manually and/or with the aid of or automation provided by electronic equipment. Although processes have been described with reference to particular embodiments, a person of ordinary skill in the art will readily appreciate that other ways of performing the acts associated with the methods may be used. For example, the order of various of the steps may be changed without departing from the scope or spirit of the method, unless described otherwise. In addition, some of the individual steps can be combined, omitted, or further subdivided into additional steps.

## Claims

1. A compound which is a salt of 1-methyl-7-(1-methyl-1H-pyrazol-4-yl)-5-(4-(trifluoromethoxy)phenyl)-1,5-dihydro-4H-imidazo[4,5-c]pyridin-4-one (Compound I): 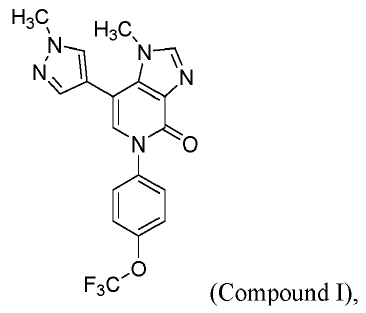 wherein the salt is selected from the group consisting of besylate, hemi-edisylate, hemi-napadisylate, mono-hydrobromide, mono-nitrate, mono-sulfate, and mono-tosylate salts of Compound I, preferably a mono-tosylate salt of Compound I.

2. The compound of claim 1, wherein the salt is mono-tosylate and **characterized by** an X-ray diffraction pattern (XRPD) when irradiated with a Cu-Kα light source substantially similar to those set forth in FIG 8.

3. The compound of claim 1 or 2, wherein the compound is mono-tosylate salt **characterized by** an X-ray diffraction pattern (XRPD) when irradiated with a Cu-Kα light source having three or more peaks selected from those at diffraction angle 2θ values of
(i) 10.92°± 0.2°, 13.28°± 0.2°, 15.36°± 0.2°, 16.94°± 0.2°, 17.74°± 0.2°, 18.20°± 0.2°, 20.51°± 0.2°, 23.21°± 0.2°, 23.86°± 0.2°, 24.73°± 0.2°, 25.69°± 0.2°, 26.68°± 0.2°, 27.63°± 0.2°, 29.12°± 0.2°, and 30.532°± 0.2°,; or
(ii) 10.92°± 0.2°, 15.36°± 0.2°, 16.94°± 0.2°, 17.74°± 0.2°, 23.21°± 0.2°, 23.86°± 0.2°, 24.73°± 0.2°, 25.69°± 0.2°, 27.63°± 0.2°, and 29.12°± 0.2°; or
(iii) 15.36°± 0.2°, 17.74°± 0.2°, 23.21°± 0.2°, 23.86°± 0.2°, and 24.73°± 0.2°.

4. The compound of any one of claims 1 to 3, wherein the compound is a mono-tosylate salt and is further **characterized by** a melt onset in a range of about 204 °C to about 207 °C.

5. The compound of any one of claims 1 to 3, wherein the compound is amorphous or crystalline.

6. A process for preparing 1-methyl-7-(1-methyl-1*H*-pyrazol-4-yl)-5-(4-(trifluoromethoxy)phenyl)-1,5-dihydro-4*H*-imidazo[4,5-c]pyridin-4-one mono-tosylate (Compound I mono-tosylate) comprising the step of:
coupling 7-bromo-1-methyl-5-(4-(trifluoromethoxy)phenyl)-1,5-dihydro-4H-imidazo[4,5-c]pyridin-4-one with 1-methyl-4-(4,4,5,5-tetramethyl-1,3,2-dioxaborolan-2-yl)-1*H-*pyrazole to yield Compound I;
contacting Compound I with *p*-toluenesulfonic acid to yield Compound I mono-tosylate.

7. The process of claim 6, comprising the step of brominating 1-methyl-5-(4-(trifluoromethoxy)phenyl)-1,5-dihydro-4H-imidazo[4,5-*c*]pyridin-4-one to yield 7-bromo-1-methyl-5-(4-(trifluoromethoxy)phenyl)-1, 5-dihydro-4H-imidazo[4, 5-*c*]pyridin-4-one.

8. The process of claim 7, comprising the step of coupling 1-methyl-1,5-dihydro-4H-imidazo[4,5-*c*]pyridin-4-one with 1-bromo-4-(trifluoromethoxy)benzene to yield 1-methyl-5-(4-(trifluoromethoxy)phenyl)-1,5-dihydro-4H-imidazo[4,5-*c*]pyridin-4-one.

9. The process of claim 8, comprising the step of condensing 2-chloro-*N*⁴-methylpyridine-3,4-diamine with formic acid to yield 1-methyl-1,5-dihydro-4H-imidazo[4,5-*c*]pyridin-4-one.

10. A granulate comprising a pharmaceutical composition comprising a Compound I mono-tosylate salt of any one of claims 1 to 5 or composition thereof, said pharmaceutical composition further comprising a pharmaceutically acceptable excipient wherein the pharmaceutically acceptable excipient comprises a binder, said excipient optionally comprising a polymeric precipitation inhibitor and said granulate optionally comprising a filler, a disintegrant, a lubricant, and a polymeric precipitation inhibitor, wherein:
(i) the binder comprises microcrystalline cellulose, carboxymethyl cellulose, or croscarmellose sodium or any combination thereof;
(ii) the filler comprises lactose, mannitol, or a combination thereof;
(iii) the disintegrant comprises croscarmellose, polyvinylpovidone, or a combination thereof;
(iv) the lubricant is selected from the group consisting of stearic acid, magnesium stearate, calcium stearate, hydrogenated vegetable oil, mineral oil, polyethylene glycol, sodium lauryl sulfate, glyceryl palmitostearate, glyceryl behenate, sodium benzoate, sodium stearyl fumarate, talc, and fumed silica, preferably magnesium stearate; and
(v) the polymeric precipitation inhibitor comprises one or more cellulosic materials, preferably hydroxypropyl methyl cellulose.

11. A pharmaceutical composition comprising the granulate of claim 10 further comprising an extragranular excipient comprising an extragranular disintegrant, extragranular lubricant, extragranular binder, or any combination thereof, and wherein
(i) the extragranular disintegrant comprises a sugar, a crosslinked polymer, a modified starch, or any combination thereof; or
(ii) the extragranular binder comprises a sugar, a starch, a sugar alcohol, a protein, a polymer, or any combination thereof; and
wherein the composition optionally further comprises a polymeric precipitation inhibitor comprising one or more cellulosic materials, preferably hydroxypropyl methylcellulose.

12. A pharmaceutical composition comprising about 30 wt% to about 40 wt% Compound I mono-tosylate, about 40 wt% to about 45 wt% binder, about 10 wt% to about 20 wt% filler, and about 0.5 wt% to about 5 wt% disintegrant.

13. A pharmaceutical composition comprising particles of a Compound I mono-tosylate salt, wherein the particles have a size distribution **characterized by** a D50 in a range of 10 µm to 60 µm, optionally wherein the D50 is in a range of 25 µm to 30 µm.

14. A pharmaceutical composition comprising particles of a Compound I mono-tosylate salt, wherein the particles have a size distribution **characterized by** a volume mean diameter D[4,3] in a range of 25 µm to 45 µm, optionally wherein the D[4,3] is in a range of 30 µm to 40 µm.

15. A pharmaceutical composition of any one of claims 13 and 14, wherein the particles are further **characterized by** D10 in a range of 1 µm to 20 µm.

16. A pharmaceutical composition of any one of claims 13 and 14 , wherein the particles are further **characterized by** D90 in a range of 50 µm to 100 µm.

17. A pharmaceutical composition comprising:
| Component | | % w/w | Mass/Tablet (mg) |
|---|---|---|---|
| Compound I mono-tosylate | | 36.06 | 288.5 |
| Microcrystalline cellulose | | 43.84 | 350.7 |
| Lactose | | 14.60 | 116.8 |
| Croscarmellose sodium | Intra-granular | 2 | 16.0 |
| | Extra-granular | 2 | 16.0 |
| Magnesium stearate | Intra-granular | 0.5 | 4.0 |
| | Extra-granular | 0.5 | 4.0 |
| HPMC | | 0.5 | 4.0 |
| Total | | 100 | 800 |

18. A process for producing a pharmaceutical composition comprising a salt of Compound I according to claim 1, the process comprising the steps of:
(a) sieving a Compound I salt according to claim 1, a binder, a filler, and a disintegrant;
(b) blending the sieved components to form a first mixture;
(c) further blending said first mixture with a lubricant to form a second mixture;
(d) compressing the second mixture;
(e) grinding the compressed second mixture;
(e) blending the ground second mixture with an extragranular disintegrant and an extragranular binder to form a third mixture;
(f) blending said third mixture with an extragranular lubricant to form a fourth mixture; and
(g) compressing said fourth mixture to form a tablet.

## Patentansprüche

1. Eine Verbindung, die ein Salz von 1-Methyl-7-(1-methyl-1*H*-pyrazol-4-yl)-5-(4-(trifluoromethoxy)phenyl)-1,5-dihydro-4*H*-imidazo[4,5-*c*]pyridin-4-on (Verbindung I) ist: 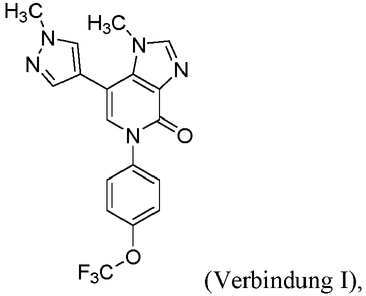 wobei das Salz ausgewählt wird aus der Gruppe bestehend aus Besylat-, Hemi-Edisylat-, Hemi-Napadisylat-, Mono-Hydrobromid-, Mono-Nitrat-, Mono-Sulfat- und Mono-Tosylat-Salzen der Verbindung I, bevorzugt ein Mono-Tosylat-Salz der Verbindung I.

2. Die Verbindung nach Anspruch 1, wobei das Salz Mono-Tosylat ist und durch ein Röntgenbeugungsmuster (XRPD) bei Bestrahlung mit einer Cu-Kα-Lichtquelle gekennzeichnet ist, das im Wesentlichen dem in FIG 8 dargestellten ähnelt.

3. Die Verbindung nach Anspruch 1 oder 2, wobei die Verbindung ein Mono-Tosylat-Salz ist, das durch ein Röntgenbeugungsmuster (XRPD) bei Bestrahlung mit einer Cu-Kα-Lichtquelle gekennzeichnet ist, das drei oder mehr Peaks aufweist, die ausgewählt werden aus denen mit Beugungswinkel 2θ-Werten von
(i) 10,92°± 0,2°, 13,28°± 0,2°, 15,36°± 0,2°, 16,94°± 0.2°, 17,74°± 0,2°, 18,20°± 0,2°, 20,51°± 0,2°, 23,21°± 0,2°, 23,86°± 0,2°, 24,73°± 0,2°, 25,69°± 0,2°, 26,68°± 0,2°, 27,63°± 0,2°, 29,12°± 0,2°, und 30,532°± 0,2°, oder
(ii) 10,92°± 0,2°, 15,36°± 0,2°, 16,94°± 0,2°, 17,74°± 0,2°, 23,21°± 0,2°, 23,86°± 0,2°, 24,73°± 0,2°, 25,69°± 0,2°, 27,63°± 0,2°, und 29,12°± 0,2°; oder
(iii) 15,36°± 0,2°, 17,74°± 0,2°, 23,21°± 0,2°, 23,86°± 0,2°, und 24,73°± 0,2°.

4. Die Verbindung nach einem der Ansprüche 1 bis 3, wobei die Verbindung ein Mono-Tosylat-Salz ist und ferner durch einen Schmelzbeginn in einem Bereich von etwa 204 °C bis etwa 207 °C gekennzeichnet ist.

5. Die Verbindung nach einem der Ansprüche 1 bis 3, wobei die Verbindung amorph oder kristallin ist.

6. Ein Verfahren zur Herstellung von 1-Methyl-7-(1-methyl-1*H*-pyrazol-4-yl)-5-(4-(trifluormethoxy)phenyl)-1,5-dihydro-4*H*-imidazo[4,5-*c*]pyridin-4-on Mono-Tosylat (Verbindung I Mono-Tosylat) umfassend den Schritt:
Kupplung von 7-Brom-1-methyl-5-(4-(trifluormethoxy)phenyl)-1,5-dihydro-4H-imidazo[4,5-c]pyridin-4-on mit 1-Methyl-4-(4,4,5,5-tetramethyl-1,3,2-dioxaborolan-2-yl)-1H-pyrazol, um Verbindung I zu erhalten;
Inkontaktbringen von Verbindung I mit p-Toluolsulfonsäure, um Verbindung I Mono-Tosylat zu erhalten.

7. Das Verfahren nach Anspruch 6, umfassend den Schritt der Bromierung von 1-Methyl-5-(4-(trifluormethoxy)phenyl)-1,5-dihydro-4H-imidazo[4,5-c]pyridin-4-on, um 7-Brom-1-methyl-5-(4-(trifluormethoxy)phenyl)-1,5-dihydro-4H-imidazo[4,5-c]pyridin-4-on zu erhalten.

8. Das Verfahren nach Anspruch 7, umfassend den Schritt der Kupplung von 1-Methyl-1,5-dihydro-4H-imidazo[4,5-c]pyridin-4-on mit 1-Brom-4-(trifluormethoxy)benzol, um 1-Methyl-5-(4-(trifluormethoxy)phenyl)-1,5-dihydro-4H-imidazo[4,5-c]pyridin-4-on zu erhalten.

9. Das Verfahren nach Anspruch 8, umfassend den Schritt der Kondensation von 2-Chlor-*N*⁴-methylpyridin-3,4-diamin mit Ameisensäure, um 1-Methyl-1,5-dihydro-4H-imidazo[4,5-c]pyridin-4-on zu erhalten.

10. Ein Granulat umfassend eine pharmazeutische Zusammensetzung umfassend ein Mono-Tosylatsalz der Verbindung I nach einem der Ansprüche 1 bis 5 oder eine Zusammensetzung davon, wobei die Zusammensetzung weiter einen pharmazeutisch verträglichen Hilfsstoff umfasst, wobei der pharmazeutisch verträgliche Hilfsstoff ein Bindemittel umfasst, wobei der Hilfsstoff optional einen polymeren Präzipitationsinhibitor umfasst, und wobei das Granulat optional einen Füllstoff, ein Sprengmittel, ein Gleitmittel und einen polymeren Präzipitationsinhibitor umfasst, wobei:
(i) das Bindemittel mikrokristalline Cellulose, Carboxymethylcellulose oder Croscarmellose-Natrium oder eine Kombination davon umfasst;
(ii) der Füllstoff Laktose, Mannitol oder eine Kombination davon umfasst;
(iii) das Sprengmittel Croscarmellose, Polyvinylpovidon oder eine Kombination davon umfasst,
(iv) das Gleitmittel ausgewählt wird aus der Gruppe bestehend aus Stearinsäure, Magnesiumstearat, Calciumstearat, hydriertem Pflanzenöl, Mineralöl, Polyethylenglykol, Natriumlaurylsulfat, Glycerylpalmitostearat, Glycerylbehenat, Natriumbenzoat, Natriumstearylfumarat, Talkum und pyrogener Kieselsäure, bevorzugt Magnesiumstearat; und
(v) der polymere Präzipitationsinhibitor ein oder mehrere Cellulosematerialien, bevorzugt Hydroxypropylmethylcellulose, umfasst.

11. Die pharmazeutische Zusammensetzung, umfassend das Granulat nach Anspruch 10, weiter umfassend einen extragranularen Hilfsstoff umfassend ein extragranulares Sprengmittel, ein extragranulares Gleitmittel, ein extragranulares Bindemittel oder eine beliebige Kombination davon, und wobei
(i) das extragranulare Sprengmittel einen Zucker, ein vernetztes Polymer, eine modifizierte Stärke oder eine beliebige Kombination davon umfasst; oder
(ii) das extragranulare Bindemittel einen Zucker, eine Stärke, einen Zuckeralkohol, ein Protein, ein Polymer oder eine beliebige Kombination davon umfasst; und
wobei die Zusammensetzung optional weiter einen polymeren Präzipitationsinhibitor umfassend ein oder mehrere Cellulosematerialien, bevorzugt Hydroxypropylmethylcellulose, umfasst.

12. Eine pharmazeutische Zusammensetzung umfassend etwa 30 Gew.-% bis etwa 40 Gew.- % Verbindung I Mono-Tosylat, etwa 40 Gew.-% bis etwa 45 Gew.-% Bindemittel, etwa 10 Gew.-% bis etwa 20 Gew.-% Füllstoff und etwa 0,5 Gew.-% bis etwa 5 Gew.-% Sprengmittel.

13. Eine pharmazeutische Zusammensetzung umfassend Partikel eines Mono-Tosylat-Salzes von Verbindung I, wobei die Partikel eine Größenverteilung aufweisen, die durch ein D50 im Bereich von 10 µm bis 60 µm gekennzeichnet ist, optional wobei der D50 im Bereich von 25 µm bis 30 µm liegt.

14. Eine pharmazeutische Zusammensetzung umfassend Partikel eines Mono-Tosylat-Salzes von Verbindung I, wobei die Partikel eine Größenverteilung aufweisen, die durch einen mittleren Volumendurchmesser D[4,3] in einem Bereich von 25 µm bis 45 µm gekennzeichnet ist, optional wobei der D[4,3] in einem Bereich von 30 µm bis 40 µm liegt.

15. Die pharmazeutische Zusammensetzung nach einem der Ansprüche 13 und 14, wobei die Partikel weiter durch einen D10 in einem Bereich von 1 µm bis 20 µm gekennzeichnet sind.

16. Die pharmazeutische Zusammensetzung nach einem der Ansprüche 13 und 14, wobei die Partikel weiter durcheinen D90 in einem Bereich von 50 µm bis 100 µm gekennzeichnet sind.

17. Eine pharmazeutische Zusammensetzung umfassend:
| Komponente | | Gew.-% | Masse/Tablette (mg) |
|---|---|---|---|
| Verbindung I Mono-Tosylat | | 36,06 | 288,5 |
| Mikrokristalline Cellulose | | 43,84 | 350,7 |
| Laktose | | 14,60 | 116,8 |
| Croscarmellose-Natrium | intragranular | 2 | 16,0 |
| | extragranular | 2 | 16,0 |
| Magnesiumstearat | intragranular | 0,5 | 4,0 |
| | extragranular | 0,5 | 4,0 |
| HPMC | | 0,5 | 4,0 |
| Gesamt | | 100 | 800 |

18. Ein Verfahren zum Herstellen einer pharmazeutischen Zusammensetzung umfassend ein Salz der Verbindung I nach Anspruch 1, wobei das Verfahren die folgenden Schritte umfasst:
(a) Sieben eines Salzes der Verbindung I nach Anspruch 1, eines Bindemittels, eines Füllstoffs und eines Sprengmittels;
(b) Vermischen der gesiebten Komponenten, um eine ersten Mischung zu bilden;
(c) weiteres Vermischen der ersten Mischung mit einem Gleitmittel, um eine zweite Mischung zu bilden;
(d) Verdichten der zweiten Mischung;
(e) Mahlen der verdichteten zweiten Mischung;
(e) Vermischen der gemahlenen zweiten Mischung mit einem extragranularen Sprengstoff und einem extragranularen Bindemittel, um eine dritte Mischung zu bilden;
(f) Vermischen der dritten Mischung mit einem extragranularen Gleitmittel, um eine vierte Mischung zu bilden; und
(g) Verdichten der vierten Mischung, um eine Tablette zu bilden.

## Revendications

1. Un composé qui est un sel de 1-méthyl-7-(1-méthyl-1*H*-pyrazol-4-yl)-5-(4-(trifluorométhoxy)phényl)-1,5-dihydro-4*H*-imidazo[4,5-*c*]pyridin-4-one (composé I) : 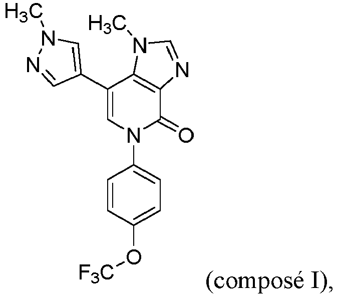 où le sel est choisi parmi le groupe comprenant les sels de bésylate, d'hémiédysilate, d'héminapadisylate, de monohydrobromure, de mononitrate, de monosulfate et de monotosylate du composé I, de préférence un sel de monotosylate du composé I.

2. Le composé de la revendication 1, où le sel est un monotosylate qui, lorsqu'il est irradié avec une source de lumière Cu-Kα, est **caractérisé par** un diagramme de diffraction des rayons X (XRPD) sensiblement similaire à ceux représentés dans la Figure 8.

3. Le composé de la revendication 1 ou 2, où le composé est un sel de monotosylate qui, lorsqu'il est irradié avec une source de lumière Cu-Kα, est **caractérisé par** un diagramme de diffraction des rayons X (XRPD) présentant au moins trois pics sélectionnés parmi ceux à des valeurs d'angle de diffraction 2θ de
(i) 10,92° ± 0,2°, 13,28° ± 0,2°, 15,36° ± 0,2°, 16,94° ± 0,2°, 17,74° ± 0,2°, 18,20° ± 0,2°, 20,51° ± 0,2°, 23,21° ± 0,2°, 23,86° ± 0,2°, 24,73° ± 0,2°, 25,69° ± 0,2°, 26,68° ± 0,2°, 27,63° ± 0,2°, 29,12° ± 0,2°, et 30,532° ± 0,2° ; ou
(ii) 10,92° ± 0,2°, 15,36° ± 0,2°, 16,94° ± 0,2°, 17,74° ± 0,2°, 23,21° ± 0,2°, 23,86° ± 0,2°, 24,73° ± 0,2°, 25,69° ± 0,2°, 27,63° ± 0,2°, et 29,12° ± 0,2° ; ou
(iii) 15,36° ± 0,2°, 17,74° ± 0,2°, 23,21° ± 0,2°, 23,86° ± 0,2° et 24,73° ± 0,2°.

4. Le composé de l'une des revendications 1 à 3, où le composé est un sel de monotosylate qui est aussi **caractérisé par** un début de fusion dans une plage d'environ 204 °C à environ 207 °C.

5. Le composé de l'une des revendications 1 à 3, où le composé est amorphe ou cristallin.

6. Un procédé de préparation du monotosylate de 1-méthyl-7-(1-méthyl-1*H*-pyrazol-4-yl)-5-(4-(trifluorométhoxy)phényl)-1,5-dihydro-4*H*-imidazo[4,5-*c*]pyridin-4-one (monotosylate du composé I) comprenant les étapes suivantes :
couplage de 7-bromo-1-méthyl-5-(4-(trifluorométhoxy)phényl)-1,5-dihydro-4*H-*imidazo[4,5-*c*]pyridin-4-one avec du 1-méthyl-4-(4,4,5,5-tétraméthyl-1,3,2-dioxaborolan-2-yl)-1*H*-pyrazole pour obtenir le composé I ;
mise en contact du composé I avec de l'acide paratoluènesulfonique pour obtenir le monotosylate du composé I.

7. Le procédé de la revendication 6, comprenant une étape de bromation de 1-méthyl-5-(4-(trifluorométhoxy)phényl)-1,5-dihydro-4*H*-imidazo[4,5-*c*]pyridin-4-one pour obtenir la 7-bromo-1-méthyl-5-(4-(trifluorométhoxy)phényl)-1,5-dihydro-4*H*-imidazo[4,5-*c*]pyridin-4-one.

8. Le procédé de la revendication 7, comprenant une étape de couplage de 1-méthyl-1,5-dihydro-4*H*-imidazo[4,5-*c*]pyridin-4-one avec du 1-bromo-4-(trifluorométhoxy)benzène pour obtenir la 1-méthyl-5-(4-(trifluorométhoxy)phényl)-1,5-dihydro-4*H*-imidazo[4,5-c]pyridin-4-one.

9. Le procédé de la revendication 8, comprenant une étape de condensation de 2-chloro-*N*⁴-méthylpyridine-3,4-diamine avec de l'acide formique pour obtenir la 1-méthyl-1,5-dihydro-4*H*-imidazo[4,5-*c*]pyridin-4-one.

10. Un granulé d'une composition pharmaceutique comprenant un sel monotosylate du composé I de l'une des revendications 1 à 5 ou d'une combinaison de celles-ci, ladite composition comprenant en outre un excipient pharmaceutiquement acceptable, lui-même comprenant un liant et éventuellement un inhibiteur de précipitation polymérique, et ledit granulé comprenant éventuellement un diluant, un délitant, un lubrifiant et un inhibiteur de précipitation polymérique, où :
(i) le liant contient de la cellulose microcristalline, de la carboxyméthylcellulose ou de la croscarmellose sodique ou toute combinaison de celles-ci ;
(ii) le diluant comprend du lactose, du mannitol ou une combinaison de ceux-ci ;
(iii) le délitant contient de la croscarmellose, de la polyvinylpovidone ou une combinaison de celles-ci ;
(iv) le lubrifiant est choisi parmi un groupe comprenant l'acide stéarique, le stéarate de magnésium, le stéarate de calcium, l'huile végétale hydrogénée, l'huile minérale, le polyéthylène glycol, le laurylsulfate de sodium, le palmitostéarate de glycéryle, le béhénate de glycéryle, le benzoate de sodium, le stéarylfumarate de sodium, le talc et la silice sublimée, de préférence le stéarate de magnésium ;
(v) l'inhibiteur de précipitation polymérique comprend une ou plusieurs matières cellulosiques, de préférence de l'hydroxypropylméthylcellulose.

11. Une composition pharmaceutique comprenant le granulé de la revendication 10 ainsi qu'un excipient extragranulaire contenant un délitant extragranulaire, un lubrifiant extragranulaire, un liant extragranulaire ou toute combinaison de ceux-ci, et où
(i) le délitant extragranulaire comprend un sucre, un polymère réticulé, un amidon modifié ou toute combinaison de ceux-ci ; ou
(ii) le liant extragranulaire comprend un sucre, un amidon, un alcool de sucre, une protéine, un polymère ou toute combinaison de ceux-ci ; et
où la composition contient éventuellement en plus un inhibiteur de précipitation polymérique comprenant une ou plusieurs matières cellulosiques, de préférence de l'hydroxypropylméthylcellulose.

12. Une composition pharmaceutique comprenant environ 30 % en poids à environ 40 % en poids de monotosylate du composé I, environ 40 % en poids à environ 45 % en poids de liant, environ 10 % en poids à environ 20 % en poids de diluant et environ 0,5 % en poids à environ 5 % en poids de délitant.

13. Une composition pharmaceutique comprenant des particules d'un sel monotosylate du composé I, où les particules ont une distribution granulométrique **caractérisée par** un D50 compris dans une plage de 10 µm à 60 µm, où D50 est éventuellement compris dans une plage de 25 µm à 30 µm.

14. Une composition pharmaceutique comprenant des particules d'un sel monotosylate du composé I, où les particules ont une distribution granulométrique **caractérisée par** un diamètre moyen en volume D[4,3] compris dans une plage de 25 µm à 45 µm, où D[4,3] est éventuellement compris dans une plage de 30 µm à 40 µm.

15. Une composition pharmaceutique de l'une des revendications 13 et 14, où les particules sont en outre **caractérisées par** un D10 compris dans une plage de 1 µm à 20 µm.

16. Une composition pharmaceutique de l'une des revendications 13 et 14, où les particules sont en outre **caractérisées par** un D90 compris dans une plage de 50 µm à 100 µm.

17. Une composition pharmaceutique comprenant :
| Composant | | % p/p | Masse/comprimé (mg) |
|---|---|---|---|
| Monotosylate du composé I | | 36,06 | 288,5 |
| Cellulose microcristalline | | 43,84 | 350,7 |
| Lactose | | 14,60 | 116,8 |
| Croscarmellose sodique | Intragranulaire | 2 | 16,0 |
| | Extragranulaire | 2 | 16,0 |
| Stéarate de magnésium | Intragranulaire | 0,5 | 4,0 |
| | Extragranulaire | 0,5 | 4,0 |
| HPMC | | 0,5 | 4,0 |
| Total | | 100 | 800 |

18. Un procédé de production d'une composition pharmaceutique comprenant un sel du composé I selon la revendication 1, le procédé incluant les étapes suivantes :
(a) tamisage d'un sel du composé I conformément à la revendication 1, d'un liant, d'un diluant et d'un délitant ;
(b) mélange des composants tamisés pour former un premier mélange ;
(c) nouveau mélange dudit premier mélange avec un lubrifiant pour obtenir un deuxième mélange ;
(d) compression du deuxième mélange ;
(e) broyage du deuxième mélange comprimé ;
(f) mélange du deuxième mélange broyé avec un délitant extragranulaire et un liant extragranulaire pour obtenir un troisième mélange ;
(g) mélange dudit troisième mélange avec un lubrifiant extragranulaire pour obtenir un quatrième mélange ;
(h) compression dudit quatrième mélange pour former un comprimé.
